# EUROPEAN PATENT APPLICATION

(11) **EP 1 020 521 A1**
(43) Date of publication of application: **19.07.2000**
(21) Application number: 99309823.5
(22) Date of filing: 07.12.1999
(51) Int. Cl.: C12N 15/12, C07K 14/705, C12N 5/10, C07K 16/28, G01N 33/53, A61K 38/17

(54) **Fas Ligand inhibitor ( FLINT ) compositions and uses thereof**

(30) Priority: 09.12.1998 US 111580 P; 07.01.1999 US 115069 P; 09.12.1998 US 111575 P
(71) Applicant: ELI LILLY AND COMPANY, Indianapolis, Indiana 46285 (US)
(72) Inventor: Song, Ho Yeong, Carmel, Indiana 46032 (US); Rosteck jr., Paul Robert, Indianapolis, Indiana 46237 (US); Su, Eric Wen, Carmel, Indiana 46032 (US)
(74) Representative: Denholm, Anna Marie

(57) **Abstract**

The present invention relates to FLINT polypeptides, including isolated nucleic acids that encode monkey FLINT, and human FLINT variants, vectors, host cells, transgenics, chimerics, and methods of making and using thereof, as well as FLINT-specific antibodies and methods.

### Annex to the description

SEQUENCE LISTING

<110> Song, Ho Yeong
      Rosteck, Paul R. Jr.
      Su, Eric W.

<120> FLINT Compositions and Uses Thereof

<130> X-12671

<140>

<141>

<160> 4

<170> PatentIn Ver. 2.0

<210> 1

<211> 900

<212> DNA

<213> Homo sapiens

<220>

<221> CDS

<222> (1)..(900)

<400> 1

atg agg gcg ctg gag ggg cca ggc ctg tcg ctg ctg tgc ctg gtg ttg    48

Met Arg Ala Leu Glu Gly Pro Gly Leu Ser Leu Leu Cys Leu Val Leu

  1               5                  10                  15

gcg ctg cct gcc ctg ctg ccg gtg ccg gct gta cgc gga gtg gca gaa 96
Ala Leu Pro Ala Leu Leu Pro Val Pro Ala Val Arg Gly Val Ala Glu
            20                  25                  30

aca ccc acc tac ccc tgg cgg gac gca gag aca ggg gag cgg ctg gtg 144
Thr Pro Thr Tyr Pro Trp Arg Asp Ala Glu Thr Gly Glu Arg Leu Val
        35                  40                  45

tgc gcc cag tgc ccc cca ggc acc ttt gtg cag cgg ccg tgc cgc cga 192
Cys Ala Gln Cys Pro Pro Gly Thr Phe Val Gln Arg Pro Cys Arg Arg
    50                  55                  60

gac agc ccc acg acg tgt ggc ccg tgt cca ccg cgc cac tac acg cag 240
Asp Ser Pro Thr Thr Cys Gly Pro Cys Pro Pro Arg His Tyr Thr Gln
65                  70                  75                  80

ttc tgg aac tac ctg gag cgc tgc cgc tac tgc aac gtc ctc tgc ggg 288
Phe Trp Asn Tyr Leu Glu Arg Cys Arg Tyr Cys Asn Val Leu Cys Gly
                85                  90                  95

gag cgt gag gag gag gca cgg gct tgc cac gcc acc cac aac cgt gcc 336
Glu Arg Glu Glu Glu Ala Arg Ala Cys His Ala Thr His Asn Arg Ala
            100                 105                 110

tgc cgc tgc cgc acc ggc ttc ttc gcg cac gct ggt ttc tgc ttg gag 384
Cys Arg Cys Arg Thr Gly Phe Phe Ala His Ala Gly Phe Cys Leu Glu
        115                 120                 125

cac gca tcg tgt cca cct ggt gcc ggc gtg att gcc ccg ggc acc ccc 432
His Ala Ser Cys Pro Pro Gly Ala Gly Val Ile Ala Pro Gly Thr Pro
    130                 135                 140

agc cag aac acg cag tgc cag ccg tgc ccc cca ggc acc ttc tca gcc 480
Ser Gln Asn Thr Gln Cys Gln Pro Cys Pro Pro Gly Thr Phe Ser Ala
145                 150                 155                 160

agc agc tcc agc tca gag cag tgc cag ccc cac cgc aac tgc acg gcc 528
Ser Ser Ser Ser Ser Glu Gln Cys Gln Pro His Arg Asn Cys Thr Ala
                165                 170                 175

ctg ggc ctg gcc ctc aat gtg cca ggc tct tcc tcc cat gac acc ctg 576
Leu Gly Leu Ala Leu Asn Val Pro Gly Ser Ser Ser His Asp Thr Leu
            180                 185                 190

tgc acc agc tgc act ggc ttc ccc ctc agc acc agg gta cca gga gct 624
Cys Thr Ser Cys Thr Gly Phe Pro Leu Ser Thr Arg Val Pro Gly Ala
        195                 200                 205

gag gag tgt gag cgt gcc gtc atc gac ttt gtg gct ttc cag gac atc 672
Glu Glu Cys Glu Arg Ala Val Ile Asp Phe Val Ala Phe Gln Asp Ile
    210                 215                 220

tcc atc aag agg ctg cag cgg ctg ctg cag gcc ctc gag gcc ccg gag 720
Ser Ile Lys Arg Leu Gln Arg Leu Leu Gln Ala Leu Glu Ala Pro Glu
225                 230                 235                 240

ggc tgg ggt ccg aca cca agg gcg ggc cgc gcg gcc ttg cag ctg aag 768
Gly Trp Gly Pro Thr Pro Arg Ala Gly Arg Ala Ala Leu Gln Leu Lys
                245                 250                 255

ctg cgt cgg cgg ctc acg gag ctc ctg ggg gcg cag gac ggg gcg ctg 816
Leu Arg Arg Arg Leu Thr Glu Leu Leu Gly Ala Gln Asp Gly Ala Leu
            260                 265                 270

ctg gtg cgg ctg ctg cag gcg ctg cgc gtg gcc agg atg ccc ggg ctg 864
Leu Val Arg Leu Leu Gln Ala Leu Arg Val Ala Arg Met Pro Gly Leu
        275                 280                 285

gag cgg agc gtc cgt gag cgc ttc ctc cct gtg cac 900
Glu Arg Ser Val Arg Glu Arg Phe Leu Pro Val His
    290                 295                 300

<210> 2
<211> 300
<212> PRT
<213> Homo sapiens

<400> 2
Met Arg Ala Leu Glu Gly Pro Gly Leu Ser Leu Leu Cys Leu Val Leu
1               5                   10                  15

Ala Leu Pro Ala Leu Leu Pro Val Pro Ala Val Arg Gly Val Ala Glu

            20                  25                  30

Thr Pro Thr Tyr Pro Trp Arg Asp Ala Glu Thr Gly Glu Arg Leu Val

        35                  40                  45

Cys Ala Gln Cys Pro Pro Gly Thr Phe Val Gln Arg Pro Cys Arg Arg

    50                  55                  60

Asp Ser Pro Thr Thr Cys Gly Pro Cys Pro Pro Arg His Tyr Thr Gln

65                  70                  75                  80

Phe Trp Asn Tyr Leu Glu Arg Cys Arg Tyr Cys Asn Val Leu Cys Gly

                85                  90                  95

Glu Arg Glu Glu Glu Ala Arg Ala Cys His Ala Thr His Asn Arg Ala

            100                 105                 110

Cys Arg Cys Arg Thr Gly Phe Phe Ala His Ala Gly Phe Cys Leu Glu

        115                 120                 125

His Ala Ser Cys Pro Pro Gly Ala Gly Val Ile Ala Pro Gly Thr Pro

    130                 135                 140

Ser Gln Asn Thr Gln Cys Gln Pro Cys Pro Pro Gly Thr Phe Ser Ala

145                 150                 155                 160

Ser Ser Ser Ser Ser Glu Gln Cys Gln Pro His Arg Asn Cys Thr Ala

                165                 170                 175

Leu Gly Leu Ala Leu Asn Val Pro Gly Ser Ser Ser His Asp Thr Leu
            180                 185                 190

Cys Thr Ser Cys Thr Gly Phe Pro Leu Ser Thr Arg Val Pro Gly Ala
        195                 200                 205

Glu Glu Cys Glu Arg Ala Val Ile Asp Phe Val Ala Phe Gln Asp Ile
    210                 215                 220

Ser Ile Lys Arg Leu Gln Arg Leu Leu Gln Ala Leu Glu Ala Pro Glu
225                 230                 235                 240

Gly Trp Gly Pro Thr Pro Arg Ala Gly Arg Ala Ala Leu Gln Leu Lys
                245                 250                 255

Leu Arg Arg Arg Leu Thr Glu Leu Leu Gly Ala Gln Asp Gly Ala Leu
            260                 265                 270

Leu Val Arg Leu Leu Gln Ala Leu Arg Val Ala Arg Met Pro Gly Leu
        275                 280                 285

Glu Arg Ser Val Arg Glu Arg Phe Leu Pro Val His
    290                 295                 300

<210> 3
<211> 900

<212> DNA

<213> monkey

<220>

<221> CDS

<222> (1)..(900)

<400> 3

atg agg gca ctg gag ggg ccg ggc ctg ctg ctg ctg tgc ctg gtg tgg    48

Met Arg Ala Leu Glu Gly Pro Gly Leu Leu Leu Leu Cys Leu Val Trp

1               5                   10                  15

gcg ctg ccc gcc ctg ctg ctg gtg ccg gcc atg cgt gga gcg aca gag    96

Ala Leu Pro Ala Leu Leu Leu Val Pro Ala Met Arg Gly Ala Thr Glu

            20                  25                  30

gca ccc act tac ccc tgg cgg gac aca gac acg ggg gag tgg ctg gtg    144

Ala Pro Thr Tyr Pro Trp Arg Asp Thr Asp Thr Gly Glu Trp Leu Val

        35                  40                  45

tgt gcc cag tgc cct cca ggc acc ttt gtg cag cag ccc tgc cgc cga    192

Cys Ala Gln Cys Pro Pro Gly Thr Phe Val Gln Gln Pro Cys Arg Arg

    50                  55                  60

gac agc ccc acg acg tgt ggc ccg tgt cct cca cgc cac tac acg cag    240

Asp Ser Pro Thr Thr Cys Gly Pro Cys Pro Pro Arg His Tyr Thr Gln

65                  70                  75                  80

ttc tgg aac tac ctg gag cgc tgc cgc tac tgc aac gtc ctc tgc ggg 288
Phe Trp Asn Tyr Leu Glu Arg Cys Arg Tyr Cys Asn Val Leu Cys Gly
                85                  90                  95

gag cgc gag gaa gag gca cgg cct tgc cac gcc acc cac aac cgc gcc 336
Glu Arg Glu Glu Glu Ala Arg Pro Cys His Ala Thr His Asn Arg Ala
            100                 105                 110

tgc cgc tgc cgc acc ggc ttc ttc gca cac gct ggt ttc tgc ctg gag 384
Cys Arg Cys Arg Thr Gly Phe Phe Ala His Ala Gly Phe Cys Leu Glu
        115                 120                 125

cat gca ttg tgt cca cct ggc acc ggc gtg atg gcc ccg ggc acc ccc 432
His Ala Leu Cys Pro Pro Gly Thr Gly Val Met Ala Pro Gly Thr Pro
    130                 135                 140

agc cag aac acg cag tgc cag ccg tgc ccc cca ggc acc ttc tca gcc 480
Ser Gln Asn Thr Gln Cys Gln Pro Cys Pro Pro Gly Thr Phe Ser Ala
145                 150                 155                 160

agc agc tcc agc tca gag cag tgc cag ccc cac cgt aac tgc acg gcc 528
Ser Ser Ser Ser Ser Glu Gln Cys Gln Pro His Arg Asn Cys Thr Ala
                165                 170                 175

ctg ggc cta gcc ctc aac gtg cca ggc tct tcc tcc cac gac gcc ctg 576
Leu Gly Leu Ala Leu Asn Val Pro Gly Ser Ser Ser His Asp Ala Leu
            180                 185                 190

tgc acc agc tgc act gcc ttc ccc ctc agc acc agg gta cca gga act 624

Cys Thr Ser Cys Thr Ala Phe Pro Leu Ser Thr Arg Val Pro Gly Thr

        195                 200                 205

gag gag tgc aag cgt gcc gtc atc gac ttc gtg gct ttc cag gat atc 672

Glu Glu Cys Lys Arg Ala Val Ile Asp Phe Val Ala Phe Gln Asp Ile

    210                 215                 220

tcc atc aag agg ttg cag cgg ctg ctg cag gcc ctt gag acc ccg ggg 720

Ser Ile Lys Arg Leu Gln Arg Leu Leu Gln Ala Leu Glu Thr Pro Gly

225                 230                 235                 240

ggc tgg ggt cca aca cca agg gcg ggc cgc gca gcc ttg cgg ctg aag 768

Gly Trp Gly Pro Thr Pro Arg Ala Gly Arg Ala Ala Leu Arg Leu Lys

                245                 250                 255

ctg caa cag cgg ctc acg gag ctc ctg gag gcg cag gac ggg gca ctt 816

Leu Gln Gln Arg Leu Thr Glu Leu Leu Glu Ala Gln Asp Gly Ala Leu

            260                 265                 270

ctg gtg cgg ctg ctg cag gca ctg cgt gtg gcc agg atg ccc ggg ctg 864

Leu Val Arg Leu Leu Gln Ala Leu Arg Val Ala Arg Met Pro Gly Leu

        275                 280                 285

gag cgg agc gtc cgt gag cgc ttc ctc cct ggg cac 900

Glu Arg Ser Val Arg Glu Arg Phe Leu Pro Gly His

    290                 295                 300

<210> 4

<211> 300

<212> PRT

<213> monkey

<400> 4

Met Arg Ala Leu Glu Gly Pro Gly Leu Leu Leu Leu Cys Leu Val Trp
1               5                   10                  15

Ala Leu Pro Ala Leu Leu Leu Val Pro Ala Met Arg Gly Ala Thr Glu
            20                  25                  30

Ala Pro Thr Tyr Pro Trp Arg Asp Thr Asp Thr Gly Glu Trp Leu Val
        35                  40                  45

Cys Ala Gln Cys Pro Pro Gly Thr Phe Val Gln Gln Pro Cys Arg Arg
    50                  55                  60

Asp Ser Pro Thr Thr Cys Gly Pro Cys Pro Pro Arg His Tyr Thr Gln
65                  70                  75                  80

Phe Trp Asn Tyr Leu Glu Arg Cys Arg Tyr Cys Asn Val Leu Cys Gly
                85                  90                  95

Glu Arg Glu Glu Glu Ala Arg Pro Cys His Ala Thr His Asn Arg Ala
            100                 105                 110

Cys Arg Cys Arg Thr Gly Phe Phe Ala His Ala Gly Phe Cys Leu Glu
        115                 120                 125
His Ala Leu Cys Pro Pro Gly Thr Gly Val Met Ala Pro Gly Thr Pro
    130                 135                 140
Ser Gln Asn Thr Gln Cys Gln Pro Cys Pro Pro Gly Thr Phe Ser Ala
145                 150                 155                 160
Ser Ser Ser Ser Ser Glu Gln Cys Gln Pro His Arg Asn Cys Thr Ala
                165                 170                 175
Leu Gly Leu Ala Leu Asn Val Pro Gly Ser Ser Ser His Asp Ala Leu
            180                 185                 190
Cys Thr Ser Cys Thr Ala Phe Pro Leu Ser Thr Arg Val Pro Gly Thr
        195                 200                 205
Glu Glu Cys Lys Arg Ala Val Ile Asp Phe Val Ala Phe Gln Asp Ile
    210                 215                 220
Ser Ile Lys Arg Leu Gln Arg Leu Leu Gln Ala Leu Glu Thr Pro Gly
225                 230                 235                 240
Gly Trp Gly Pro Thr Pro Arg Ala Gly Arg Ala Ala Leu Arg Leu Lys
                245                 250                 255
Leu Gln Gln Arg Leu Thr Glu Leu Leu Glu Ala Gln Asp Gly Ala Leu

            260                 265                 270

Leu Val Arg Leu Leu Gln Ala Leu Arg Val Ala Arg Met Pro Gly Leu

        275                 280                 285

Glu Arg Ser Val Arg Glu Arg Phe Leu Pro Gly His

    290                 295                 300

## Description

This application claims the benefit of U.S. Provisional Application Nos. 60/111,580, filed December 9, 1998; 60/115,069, filed January 7, 1998; and 60/111,575, filed December 9, 1998.

The present invention relates to compounds and compositions comprising Fas Ligand inhibitor ("FLINT") and related polypeptides, nucleic acids, host cells, transgenics, chimerics, antibodies, compositions, and methods of making and using thereof.

Apoptosis (i.e. programmed cell death) plays a central role in development and homeostasis. Cells die by apoptosis in the developing embryo and in the adult animal. Because the physiological role of apoptosis is crucial, aberration of this process can be detrimental. For example, unscheduled apoptosis of certain brain neurons contributes to disorders such as Alzheimer's and Parkinson's disease, whereas the failure of dividing cells to initiate apoptosis after sustaining severe DNA damage contributes to cancer.

Survival signals from the cell's environment and internal sensors for cellular integrity normally keep a cell's apoptotic machinery in check. In the event that a cell loses contact with its surroundings or sustains irreparable damage, the cell initiates apoptosis. Mammals have evolved yet another mechanism that enables the organism actively to direct individual cells to self-destruct. This kind of "instructive" apoptosis is important especially in the immune system. Death receptors-cell surface receptors that transmit apoptosis signals initiated by specific "death ligands" - play a central role in instructive role apoptosis. These receptors can activate death caspases within seconds of ligands binding, causing an apoptotic demise of the cell within hours.

Death receptors belongs to the tumor necrosis factor (TNF) receptor gene superfamily, which is defined by similar, cysteine-rich extracellular domains. The death receptors contain in addition a homologous cytoplasmic sequence termed the "death domain".

Fas (also called CD95 or Apo1) is a knownn death receptor. Fas and Fas ligand (FasL) play an important role in apoptosis. Fas L is a homotrimeric molecule that binds three Fas molecules. Because death domains have a propensity to associate with one another, Fas ligation leads to clustering of the receptors' death domains. An adapter protein called FADD (Fas-associated death domain; also called Mort1) then binds through its own death domain to the clustered receptor death domains. FADD also contains a "death effector domain" that binds to an analogous domain repeated in tandem within the zymogen form of caspase-8 (also called FLICE, or MACH). Upon recruitment by FADD, caspase-8 oligomerization drives its activation through self-cleavage. Caspase-8 then activates downstream effector caspases committing the cell to apoptosis. Ashkenazi A., et al. "Death Receptors: Signaling and Modulation, Science, 281, 1305-1308 (1998).

FLINT has been found to bind to FasL. Thus, by binding FasL, FLINT can interfere with FasL binding to Fas receptor to modulate apoptosis. Still further, by antagonizing FasL, FLINT can modulate the destruction of cells caused by neutrophils which have been activated by FasL.

Accordingly, there is a need to provide FLINT and FLINT related polypeptides, nucleic acids, host cells, transgenics, chimerics, as well as methods of making and using thereof.

The present invention provides isolated nucleic acids, encoded FLINT polypeptides, including specified fragments and variants thereof, FLINT compositions, probes, primers, vectors, host cells, antibodies, transgenics, chimerics and methods of making and using thereof, as described and enabled herein.

The present invention provides, in one aspect, isolated nucleic acid molecules comprising or complementary to a polynucleotide encoding monkey FLINT polypeptide (SEQ ID NO:4), fragments or specified variants thereof.

The present invention further provides recombinant vectors, comprising said isolated monkey FLINT nucleic acid molecules of the present invention, host cells containing such nucleic acids and/or recombinant vectors, as well as methods of making and/or using such nucleic acid, vectors and/or host cells.

The present invention also provides methods of making or using such nucleic acids, vectors and/or host cells, such as but not limited to, using them for the production of monkey FLINT nucleic acids and/or polypeptides by known recombinant, synthetic and/or purification techniques, based on the teaching and guidance presented herein in combination with what is known in the art.

The present invention also provides an isolated monkey FLINT polypeptide, comprising at least one fragment, domain or specified variant of at least 90-100% of the contiguous amino acids of SEQ ID NO:4.

The present invention also provides an isolated monkey FLINT polypeptide as described herein, wherein the polypeptide has at least one activity, such as, but not limited to a Fas ligand binding assay as described herein.

The present invention also provides a composition comprising an isolated monkey FLINT nucleic acid and/or polypeptide as described herein and a carrier or diluent. The carrier or diluent can optionally be pharmaceutically acceptable, according to known methods.

The present invention also provides an isolated nucleic acid probe, primer or fragment, as described herein, wherein the nucleic acid comprises a polynucleotide of at least 10 nucleotides, corresponding or complementary to at least 10 nucleotides of SEQ ID NO:3.

The present invention also provides a recombinant vector comprising an isolated monkey FLINT nucleic acid as described herein.

The present invention also provides a host cell, comprising an isolated monkey FLINT nucleic acid as described herein.

The present invention also provides a method for constructing a recombinant host cell that expresses a monkey FLINT polypeptide, comprising introducing into the host cell a monkey FLINT nucleic acid in replicable form.

The present invention also relates to a recombinant host cell provided by a method as described herein.

The present invention also provides a method for expressing at least one monkey FLINT polypeptide in a recombinant host cell, comprising culturing a recombinant host cell as described herein under conditions wherein at least one monkey FLINT polypeptide is expressed in detectable or recoverable amounts.

The present invention also provides an isolated monkey FLINT polypeptide (SEQ ID NO:4) produced by a recombinant, synthetic, and/or any suitable purification method as described herein and/or as known in the art.

The present invention also provides a monkey FLINT antibody or fragment, comprising a polyclonal and/or monoclonal antibody or fragment that specifically binds at least one epitope specific to at least one monkey FLINT polypeptide as described herein.

The present invention also provides a method for producing a monkey FLINT antibody or antibody fragment, comprising generating the antibody or fragment that binds at least one epitope that is specific to an isolated monkey FLINT polypeptide as described herein, the generating done by knowing recombinant, synthetic and/or hybridoma methods.

The present invention also provides a monkey FLINT antibody or fragment produced by a method as described herein or as known in the art.

The present invention also provides a method for identifying compounds that bind a monkey FLINT polypeptide, comprising
a) admixing at least one isolated monkey FLINT polypeptide as described herein with a test compound or composition; and
b) detecting at least one binding interaction between the polypeptide and the compound or composition, optionally further comprising detecting a change in biological activity, such as a reduction or increase.

The present invention also contemplates structurally and/or functionally equivalent variants of human FLINT (SEQ ID NO:2) said variants comprising single and/or multiple amino acid substitutions in SEQ ID NO:2, wherein:
a) Ser at position 10 is alternatively substituted by Thr, Leu, Val, Ile, Gly, or Ala;
b) Leu at position 16 is alternatively substituted by Ile, Val, Ala, Trp or Gly;
c) Pro at position 23 is alternatively substituted by Leu, Ile, Val, Ala, or Gly;
d) Val at position 27 is alternatively substituted by Gly, Ala, Leu, Met or Ile;
e) Val at position 30 is alternatively substituted by Leu, Ile, Val, Ala, or Gly;
f) Ala at position 31 is alternatively substituted by Gly, Val, Leu, Thr or Ile;
g) Thr at position 33 is alternatively substituted by Ser, Ala, Gly, Leu, Val, or Ile;
h) Ala at position 41 is alternatively substituted by Thr, Gly, Val, Leu, or Ile;
i) Glu at position 42 is alternatively substituted by Asn, Asp, or Gln;
j) Arg at position 46 is alternatively substituted by His, Trp, or Lys;
k) Arg at position 60 is alternatively substituted by His, Gln, or Lys;
l) Ala at position 104 is alternatively substituted by Gly, Val, Leu, Pro, or Ile;
m) Ser at position 131 is alternatively substituted by Thr, Ile; Leu, Ala, or Gly;
n) Ala at position 136 is alternatively substituted by Gly, Val, Leu, Thr, or Ile;
o) Ile at position 139 is alternatively substituted by Leu, Gly, Ala, Met, or Val;
p) Thr at position 191 is alternatively substituted by Ser, Ala, Gly, Leu, Ile, or Val;
q) Gly at position 198 is alternatively substituted by Ala, Val, Ile, or Leu;
r) Ala at position 208 is alternatively substituted by Gly, Leu, Val, Thr, or Ile;
s) Glu at position 212 is alternatively substituted by Asp, Asn, Lys, or Gln;
t) Ala at position 238 is alternatively substituted by Gly, Leu, Val, Thr, or Ile;
u) Gln at position 254 is alternatively substituted by Asn, Asp, Glu, Arg, Lys, or His;
v) Arg at position 258 is alternatively substituted by Lys, His, Gln, Asn, Asp, or Glu;
w) Arg at position 259 is alternatively substituted by Lys, His, Gln, Asn, Asp, or Glu;
x) Gly at position 266 is alternatively substituted by Ala, Val, Leu, Ile, Asp, Glu, Asn, or Gln; and
y) Val at position 299 is alternatively substituted by Leu, Ile, Ala, or Gly.

The present invention also contemplates structurally and/or functionally equivalent variants of human FLINT (SEQ ID NO:2) said variants comprising single amino acid substitutions in SEQ ID NO:2, wherein:
a) Ser at position 10 is substituted by Leu;
b) Leu at position 16 is substituted by Trp;
c) Pro at position 23 is substituted by Leu;
d) Val at position 27 is substituted by Met;
e) Val at position 30 is substituted by Ala;
f) Ala at position 31 is substituted by Thr;
g) Thr at position 33 is substituted by Ala;
h) Ala at position 41 is substituted by Thr;
i) Glu at position 42 is substituted by Asp;
j) Arg at position 46 is substituted by Trp;
k) Arg at position 60 is substituted by Gln;
l) Ala at position 104 is substituted by Pro;
m) Ser at position 131 is substituted by Leu;
n) Ala at position 136 is substituted by Thr;
o) Ile at position 139 is substituted by Met;
p) Thr at position 191 is substituted by Ala;
q) Gly at position 198 is substituted by Ala;
r) Ala at position 208 is substituted by Thr;
s) Glu at position 212 is substituted by Lys;
t) Ala at position 238 is substituted by Thr;
u) Gln at position 254 is substituted by Arg;
v) Arg at position 258 is substituted by Gln;
w) Arg at position 259 is substituted by Gln;
x) Gly at position 266 is substituted by Glu; and
y) Val at position 299 is substituted by Gly.

In another embodiment, the present invention relates to therapeutic and clinical uses of a FLINT polypeptide of the present invention to prevent or treat a disease or condition in a mammal in need of such prevention or treatment.

In another embodiment, the present invention relates to a pharmaceutical formulation comprising a FLINT polypeptide of the present invention.

The present invention provides isolated, recombinant and/or synthetic FLINT proteins and nucleic acid molecules relating to monkey FLINT and variants of human FLINT in which one or more amino acids of SEQ ID NO:2 are substituted by the corresponding residue(s), or conservative substitutions thereof, of monkey FLINT (SEQ ID NO:4). Such polypeptides comprise specific full length sequences, fragments and specified variants thereof, and methods of making and using said nucleic acids and polypeptides. A monkey FLINT polypeptide of the invention comprises at least one fragment, domain, and/or specified variant as a portion or fragment of a monkey FLINT protein as described herein.

### Utility

The present invention also provides at least one utility by providing isolated nucleic acid comprising polynucleotides of sufficient length and complementarity to a monkey FLINT nucleic acid for use as probes or amplification primers in the detection, quantitation, or isolation of gene sequences or transcripts. For example, isolated nucleic acids of the present invention can be used as probes for detecting deficiencies in the level of mRNA, in screens for detection of mutations in at least one monkey FLINT gene (e.g., substitutions, deletions, or additions), or for monitoring upregulation of expression of said gene, or changes in biological activity as described herein in screening assays of compounds, and/or for detection of any number of allelic variants (polymorphisms or isoforms) of the gene.

The isolated nucleic acids of the present invention can also be used for recombinant expression of monkey FLINT polypeptides, or for use as immunogens in the preparation and/or screening of antibodies. The isolated nucleic acids of the present invention can also be employed for use in sense or antisense suppression of one or more monkey FLINT genes or nucleic acids, in a host cell, or tissue *in vivo* or *in vitro.* Attachment of chemical agents which bind, intercalate, cleave and/or crosslink to the isolated nucleic acids of the present invention can also be used to modulate transcription or translation of at least one nucleic acid disclosed herein.

### Citations

All publications or patents cited herein are entirely incorporated herein by reference as they show the state of the art at the time of the present invention and provide description and enablement of the present invention. Publications refer to scientific, patent publication or any other information available in any media format, including all recorded, electronic or printed formats. Included are the following citations: Ausubel, et al., ed., Current Protocols in Molecular Biology, Greene Publishing, NY, NY (1987-1998); Sambrook, et al., Molecular Cloning: A Laboratory Manual, 2^{nd} Edition, Cold Spring Harbor, NY (1989); Harlow and Lane, Antibodies, a Laboratory Manual, Cold Spring Harbor, NY (1989); Colligan, et al., eds., Current Protocols in Immunology, Greene Publishing, NY (1994-1998).

### Definitions

A "polynucleotide" comprises at least 10-20 nucleotides of a nucleic acid (RNA, DNA or combination thereof), provided by any means, such as synthetic, recombinant isolation or purification method steps.

The terms "complementary" or "complementarity" as used herein refer to the capacity of purine, pyrimidine, synthetic or modified nucleotides to associate by partial or complete complementarity through hydrogen or other bonding to form partial or complete double- or triple-stranded nucleic acid molecules. The following base pairs occur by complete complementarity: (i) guanine (G) and cytosine (C); (ii) adenine (A) and thymine (T); and adenine (A) and uracil (U). "Partial complementarity" refers to association of two or more bases by one or more hydrogen bonds or attraction that is less than the complete complementarity as described above. Partial or complete complementarity can occur between any two nucleotides, including naturally occurring or modified bases, e.g., as listed in 37 CFR § 1.822. All such nucleotides are included in polynucleotides of the invention as described herein.

The term "FLINT" or "FLINT polypeptide" as used herein refers generally to monkey FLINT (SEQ ID NO:4) and human FLINT (SEQ ID NO:4) proteins, nucleic acids encoding same, and protein and nucleic acid variants thereof, said proteins having biological activity and mechanistic action in vivo, and/or in vitro that may include inhibition of apoptosis, binding to FasL, and/or binding to LIGHT.

The term "FLINT variants" as used herein refers in particular but not exclusively to variants of human FLINT (SEQ ID NO:2) in which one or more substitutions in the amino acid sequence of SEQ ID NO:2 are made replacing the naturally occurring residue(s) with residue(s) from monkey FLINT (SEQ ID NO:4). As the skilled artisan recognizes, protein function generally is maintained following conservative amino acid substitution(s), and/or substitution by one or more residues occurring in a closely related homolog. Such substitution(s) may be made in any region of FLINT, preferably in region(s) D1 through D4.

The term "fusion protein" denotes a hybrid protein molecule not found in nature comprising a translational fusion or enzymatic fusion in which two or more different proteins or fragments thereof are covalently linked on a single polypeptide chain. The term "polypeptide" also includes such fusion proteins.

"Host cell" refers to any eucaryotic, procaryotic, or fusion or other cell or pseudo cell or membrane-containing construct that is suitable for propagating and/or expressing an isolated nucleic acid that is introduced into a host cell by any suitable means known in the art (e.g., but not limited to, transformation or transfection, or the like), or induced to express an endogenous nucleic acid encoding a monkey FLINT polypeptide according to the present invention. The cell can be part of a tissue or organism, isolated in culture or in any other suitable form.

The term "hybridization" as used herein refers to a process in which a partially or completely single-stranded nucleic acid molecule joins with a complementary strand through nucleotide base pairing. Hybridization can occur under conditions of low, moderate or high stringency, with high stringency preferred. The degree of hybridization depends upon, for example, the degree of homology, the stringency conditions, and the length of hybridizing strands as known in the art.

By "isolated" nucleic acid molecule(s) is intended a nucleic acid molecule, DNA, RNA, or both which has been removed from its native or naturally occurring environment. For example, recombinant nucleic acid molecules contained or generated in culture, a vector and/or a host cell are considered isolated for the purposes of the present invention. Further examples of isolated nucleic acid molecules include recombinant nucleic acid molecules maintained in heterologous host cells or purified (partially or substantially) nucleic acid molecules in solution. Isolated RNA molecules include in vivo or in vitro RNA transcripts of the nucleic acid molecules of the present invention. Isolated nucleic acid molecules according to the present invention further include such molecules produced synthetically, purified from or provided in cells containing such nucleic acids, where the nucleic acid exists in other than a naturally occurring form, quantitatively or qualitatively.

"Isolated" used in reference to at least one polypeptide of the invention describes a state of isolation such that the peptide or polypeptide is not in a naturally occurring form and/or has been purified to remove at least some portion of cellular or non-cellular molecules with which the protein is naturally associated. However, "isolated" may include the addition of other functional or structural polypeptides for a specific purpose, where the other peptide may occur naturally associated with at least one polypeptide of the present invention, but for which the resulting compound or composition does not exist naturally.

A "nucleic acid probe," "oligonucleotide probe," or "probe" as used herein comprises at least one detectably labeled or unlabeled nucleic acid which hybridizes under specified hybridization conditions with at least one other nucleic acid. This term also refers to a single- or partially double-stranded nucleic acid, oligonucleotide or polynucleotide that will associate with a complementary or partially complementary target nucleic acid to form at least a partially double-stranded nucleic acid molecule. A nucleic acid probe may be an oligonucleotide or a nucleotide polymer. A probe can optionally contain a detectable moiety which may be attached to the end(s) of the probe or be internal to the sequence of the probe, termed a "detectable probe" or "detectable nucleic acid probe."

A "primer" is a nucleic acid fragment or oligonucleotide which functions as an initiating substrate for enzymatic or synthetic elongation of, for example, a nucleic acid molecule, e.g., using an amplification reaction, such as, but not limited to, a polymerase chain reaction (PCR), as known in the art.

The term "stringency" refers to hybridization conditions for nucleic acids in solution. High stringency conditions disfavor non-homologous base pairing. Low stringency conditions have much less of this effect. Stringency may be altered, for example, by changes in temperature and/or salt concentration, or other conditions, as well known in the art.

A non-limiting example of "high stringency" conditions includes, for example, (a) a temperature of about 42°C , a formamide concentration of about < 20%, and a low salt (SSC) concentration, or, alternatively, a temperature of about 65° C, or less, and a low salt (SSPE) concentration; (b) hybridization in 0.5 M NaHPO4, 7% sodium dodecyl sulfate (SDS), 1 mM EDTA at 65°C (See, e.g., Ausubel, et al., ed., Current Protocols in Molecular Biology, 1987-1998, Wiley Interscience, New York, at §2.10.3). "SSC" comprises a hybridization and wash solution. A stock 20X SSC solution contains 3M sodium chloride, 0.3M sodium citrate, pH 7.0. "SSPE" comprises a hybridization and wash solution. A 1X SSPE solution contains 180 mM NaCl, 9mM Na2HPO4, 0.9 mM NaH2PO4 and 1 mM EDTA, pH 7.4.

The term "vector" as used herein refers to a nucleic acid compound used for introducing exogenous or endogenous nucleic acid into host cells. A vector comprises a nucleotide sequence which may encode one or more polypeptide molecules. Plasmids, cosmids, viruses and bacteriophages, in a natural state or which have undergone recombinant engineering, are non-limiting examples of commonly used vectors to provide recombinant vectors comprising at least one desired isolated nucleic acid molecule.

Using the information provided herein, such as the nucleotide sequences encoding at least 90-100% of the contiguous amino acids of at least one of SEQ ID NO:2, specified fragments or variants thereof, or a deposited vector comprising at least one of these sequences, a nucleic acid molecule of the present invention encoding a monkey FLINT polypeptide can be obtained using well-known methods.

Nucleic acid molecules of the present invention can be in the form of RNA, such as mRNA, hnRNA, tRNA or any other form, or in the form of DNA, including, but not limited to, cDNA and genomic DNA obtained by cloning or produced synthetically, or any combination thereof. The DNA can be triple-stranded, double-stranded or single-stranded, or any combination thereof. Any portion of at least one strand of the DNA or RNA can be the coding strand, also known as the sense strand, or it can be the non-coding strand, also referred to as the anti-sense strand.

Isolated nucleic acid molecules of the present invention include nucleic acid molecules comprising an open reading frame (ORF) shown in at least one of SEQ ID NO:1, ; nucleic acid molecules comprising the coding sequence for a monkey FLINT polypeptide; and nucleic acid molecules which comprise a nucleotide sequence substantially different from those described above but which, due to the degeneracy of the genetic code, still encode at least one monkey FLINT polypeptide as described herein. Of course, the genetic code is well known in the art. Thus, it would be routine for one skilled in the art to generate such degenerate nucleic acid variants that code for specific monkey FLINT polypeptides of the present invention. See, e.g., Ausubel, et al., *supra,* and such nucleic acid variants are included in the present invention.

In a further embodiment, nucleic acid molecules are provided encoding the mature monkey FLINT polypeptide or the full-length monkey FLINT polypeptide lacking the N-terminal methionine. The invention also provides an isolated nucleic acid molecule having the nucleotide sequence shown in at least one of SEQ ID NO:1, or the nucleotide sequence of the monkey FLINT cDNA contained in at least one of the above-described deposited clones listed herein, or a nucleic acid molecule having a sequence complementary thereto. Such isolated molecules, particularly nucleic acid molecules, are useful as probes for gene mapping by in situ hybridization with chromosomes, and for detecting transcription, translation and/or expression of the monkey FLINT gene in human tissue, for instance, by Northern blot analysis for mRNA detection.

Unless otherwise indicated, all nucleotide sequences identified by sequencing a nucleic acid molecule herein can be or were identified using an automated nucleic acid sequencer, and all amino acid sequences of polypeptides encoded by nucleic acid molecules identified herein can be or were identified by codon correspondence or by translation of a nucleic acid sequence identified using method steps as described herein or as known in the art. Therefore, as is well known in the art that for any nucleic acid sequence identified by this automated approach, any nucleotide sequence identified herein may contain some errors which are reproducibly correctable by resequencing based upon an available or a deposited vector or host cell containing the nucleic acid molecule using well-known methods.

Nucleotide sequences identified by automation are typically at least about 95% to at least about 99.999% identical to the actual nucleotide sequence of the sequenced nucleic acid molecule. The actual sequence can be more precisely identified by other approaches including manual nucleic acid sequencing methods well known in the art. As is also known in the art, a single insertion or deletion in an identified nucleotide sequence compared to the actual sequence will cause a frame shift in translation of the nucleotide sequence such that the identified amino acid sequence encoded by an identified nucleotide sequence will be completely different from the amino acid sequence actually encoded by the sequenced nucleic acid molecule, beginning at the point of such an insertion or deletion.

### Nucleic Acid Fragments

The present invention is further directed to fragments of the isolated nucleic acid molecules described herein. By a fragment of an isolated nucleic acid molecule is meant a molecule having at least 10 nucleotides of a nucleotide sequence of a deposited cDNA or a nucleotide sequence shown in at least one of SEQ ID NO:1, and is intended to mean fragments at least about 10 nucleotides, and at least about 40 nucleotides in length, which are useful, *inter alia* as diagnostic probes and primers as described herein. Of course, larger fragments such as at least about 50, 100, 120, 200, 500 or more nucleotides in length, are also useful according to the present invention as are fragments corresponding to most, if not all, of the nucleotide sequence (or the deposited cDNA) as shown at least one of SEQ ID NO:1. By a fragment at least 10 nucleotides in length, for example, is intended fragments which include 10 or more contiguous nucleotides from the nucleotide sequence of the deposited cDNA or the nucleotide sequence as shown in SEQ ID NO:1, or consensus sequences thereof, as determined by methods known in the art.

Such nucleotide fragments are useful according to the present invention for screening DNA sequences that code for one or more fragments of a monkey FLINT polypeptide as described herein. Such screening, as a non-limiting example can include the use of so-called "DNA chips" for screening DNA sequences of the present invention of varying lengths, as described, e.g., in U.S. Patent Nos. 5,631,734, 5,624,711, 5,744,305, 5,770,456, 5,770,722, 5,675,443, 5,695,940, 5,710,000, 5,733,729, which are entirely incorporated herein by reference.

As indicated, nucleic acid molecules of the present invention which comprise a nucleic acid encoding a monkey FLINT polypeptide can include, but are not limited to, those encoding the amino acid sequence of the mature polypeptide, by itself; the coding sequence for the mature polypeptide and additional sequences, such as the coding sequence of at least one signal leader or fusion peptide or of the mature polypeptide, with or without the aforementioned additional coding sequences, such as at least one intron, together with additional, non-coding sequences, including but not limited to, introns and non-coding 5' and 3' sequences, such as the transcribed, non-translated sequences that play a role in transcription, mRNA processing, including splicing and polyadenylation signals (for example - ribosome binding and stability of mRNA); an additional coding sequence which codes for additional amino acids, such as those which provide additional functionalities. Thus, the sequence encoding a polypeptide can be fused to a marker sequence, such as a sequence encoding a peptide that facilitates purification of the fused polypeptide.

Preferred nucleic acid fragments of the present invention also include nucleic acid molecules encoding epitope-bearing portions of a monkey FLINT polypeptide.

### Oligonucleotide and Polynucleotide Probes and/or Primers

In another aspect, the invention provides a polynucleotide (either DNA or RNA) that comprises at least about 20 nt, still more preferably at least about 30 nt, and even more preferably at least about 30-2000 nt of a nucleic acid molecule described herein. These are useful as diagnostic probes and primers as discussed above and in more detail below.

By a portion of a polynucleotide of "at least 10 nt in length," for example, is intended 10 or more contiguous nucleotides from the nucleotide sequence of the reference polynucleotide (e.g., at least one deposited nucleic acid or at least one nucleotide sequence as shown in at least one of SEQ ID NO:1, ).

Of course, a polynucleotide which hybridizes only to a poly A sequence (such as the 3' terminal poly(A) of a monkey FLINT cDNA shown in at least one of SEQ ID NO:1, or to a complementary stretch of T (or U) resides, would not be included in a probe of the invention, since such a polynucleotide would hybridize to any nucleic acid molecule containing a poly (A) stretch or the complement thereof (e.g., practically any double-stranded cDNA clone).

The present invention also provides subsequences of full-length nucleic acids. Any number of subsequences can be obtained by reference to at least one of SEQ ID NO:1, or a complementary sequence, and using primers which selectively amplify, under stringent conditions to: at least two sites to the polynucleotides of the present invention, or to two sites within the nucleic acid which flank and comprise a polynucleotide of the present invention, or to a site within a polynucleotide of the present invention and a site within the nucleic acid which comprises it. A variety of methods for obtaining 5' and/or 3' ends is well known in the art. See, e.g., RACE (Rapid Amplification of Complementary Ends) as described in M. A. Frohman, PCR Protocols: A Guide to Methods and Applications, M. A. Innis, D. H. Gelfand, J. J. Sninsky, T. J. White, Eds., Academic Press, Inc., San Diego, CA, pp. 28-38 (1990); see also, U.S. Patent No. 5,470,722, and Ausubel, et al., Current Protocols in Molecular Biology, Unit 15.6, Eds., Greene Publishing and Wiley-Interscience, New York (1989-1998). Thus, the present invention provides monkey FLINT polynucleotides having the sequence of the monkey FLINT gene, nuclear transcript, cDNA, or complementary sequences and/or subsequences thereof.

Primer sequences can be obtained by reference to a contiguous subsequence of a polynucleotide of the present invention. Primers are chosen to selectively hybridize, under PCR amplification conditions, to a polynucleotide of the present invention in an amplification mixture comprising a genomic and/or cDNA library from the same species. Generally, the primers are complementary to a subsequence of the amplified nucleic acid. In some embodiments, the primers will be constructed to anneal at their 5' terminal ends to the codon encoding the carboxy or amino terminal amino acid residue (or the complements thereof) of the polynucleotides of the present invention. The primer length in nucleotides is selected from the group of integers consisting of from at least 15 to 50. Thus, the primers can be at least 15, 18, 20, 25, 30, 40, or 50 nucleotides in length or any range or value therein. A non-annealing sequence at the 5' end of the primer (a "tail") can be added, for example, to introduce a cloning site at the terminal ends of the amplified DNA.

The amplification primers may optionally be elongated in the 3' direction with additional contiguous or complementary nucleotides from the polynucleotide sequences, such as at least one of SEQ ID NO:1, from which they are derived. The number of nucleotides by which the primers can be elongated is selected from the group of integers consisting of from at least 1 to at least 25. Thus, for example, the primers can be elongated with an additional 1, 5, 10, or 15 nucleotides or any range or value therein. Those of skill will recognize that a lengthened primer sequence can be employed to increase specificity of binding (i.e., annealing) to a target sequence, or to add useful sequences, such as links or restriction sites.

The amplification products can be translated using expression systems well known to those of skill in the art and as discussed, infra. The resulting translation products can be confirmed as polypeptides of the present invention by, for example, assaying for the appropriate catalytic activity (e.g., specific activity and/or substrate specificity), or verifying the presence of one or more linear epitopes which are specific to a polypeptide of the present invention. Methods for protein synthesis from PCR derived templates are known in the art and available commercially. See, e.g., Amersham Life Sciences, Inc., Catalog '97, p. 354.

The present invention is intended to provide novel isolated nucleic acids that hybridize under selective hybridization conditions to a polynucleotide disclosed herein, e.g., SEQ ID NO:1 and/or SEQ ID NO:3, said hybridizing nucleic acids also encoding polypeptides having FLINT activity including, for example, the ability to suppress apoptosis in vivo and/or in vitro. For example, polynucleotides of the present invention can be used to identify, isolate, or amplify partial or full-length clones in a deposited library. In some embodiments, the polynucleotides are genomic or cDNA sequences isolated, or otherwise complementary to, a cDNA from a human or mammalian nucleic acid library.

Preferably, the cDNA library comprises at least 80% full-length sequences, preferably at least 85% or 90% full-length sequences, and more preferably at least 95% full-length sequences. The cDNA libraries can be normalized to increase the representation of rare sequences. Low stringency hybridization conditions are typically, but not exclusively, employed with sequences having a reduced sequence identity relative to complementary sequences. Moderate and high stringency conditions can optionally be employed for sequences of greater identity. Low stringency conditions allow selective hybridization of sequences having about 70% sequence identity and can be employed to identify orthologous or paralogous sequences.

Optionally, polynucleotides of this invention will encode an epitope of a polypeptide encoded by the polynucleotides described herein. The polynucleotides of this invention embrace nucleic acid sequences which can be employed for selective hybridization to a polynucleotide encoding a polypeptide of the present invention.

Screening polypeptides for specific binding to antibodies or fragments can be conveniently achieved using peptide display libraries. This method involves the screening of large collections of peptides for individual members having the desired function or structure. Antibody screening of peptide display libraries is well known in the art. The displayed peptide sequences can be from 3 to 5000 or more amino acids in length, frequently from 5-100 amino acids long, and often from about 8 to 15 amino acids long. In addition to direct chemical synthetic methods for generating peptide libraries, several recombinant DNA methods have been described. One type involves the display of a peptide sequence on the surface of a bacteriophage or cell. Each bacteriophage or cell contains the nucleotide sequence encoding the particular displayed peptide sequence. Such methods are described in PCT Patent Publication Nos. 91/17271, 91/18980, 91/19818, and 93/08278. Other systems for generating libraries of peptides have aspects of both in vitro chemical synthesis and recombinant methods. See, PCT Patent Publication Nos. 92/05258, 92/14843, and 96/19256. See also, U.S. Patent Nos. 5,658,754; and 5,643,768. Peptide display libraries, vector, and screening kits are commercially available from such suppliers as Invitrogen (Carlsbad, CA). (See, e.g., Ausubel, *supra;* or Sambrook, *supra) .*

As indicated above, the present invention provides isolated nucleic acids comprising monkey FLINT polynucleotides, wherein the polynucleotides are complementary to the polynucleotides described herein, above. As those of skill in the art will recognize, complementary sequences base-pair throughout the entirety of their length with such polynucleotides (i.e., have 100% sequence identity over their entire length). Complementary bases associate through hydrogen bonding in double-stranded nucleic acids. For example, the following base pairs are complementary: guanine and cytosine; adenine and thymine; and adenine and uracil. (See, e.g., Ausubel, *supra;* or Sambrook, *supra)*

The isolated nucleic acids of the present invention can be made using (a) standard recombinant methods, (b) synthetic techniques, (c) purification techniques, or combinations thereof, as well known in the art.

The nucleic acids may conveniently comprise sequences in addition to a polynucleotide of the present invention. For example, a multi-cloning site comprising one or more endonuclease restriction sites may be inserted into the nucleic acid to aid in isolation of the polynucleotide. Also, translatable sequences may be inserted to aid in the isolation of the translated polynucleotide of the present invention. For example, a hexa-histidine marker sequence provides a convenient means to purify the proteins of the present invention. The nucleic acid of the present invention - excluding the polynucleotide sequence - is optionally a vector, adapter, or linker for cloning and/or expression of a polynucleotide of the present invention.

Additional sequences may be added to such cloning and/or expression sequences to optimize their function in cloning and/or expression, to aid in isolation of the polynucleotide, or to improve the introduction of the polynucleotide into a cell. Typically, the length of a nucleic acid of the present invention less the length of its polynucleotide of the present invention is less than 20 kilobase pairs, often less than 15 kb, and frequently less than 10 kb. Use of cloning vectors, expression vectors, adapters, and linkers is well known in the art. (See, e.g., Ausubel, *supra;* or Sambrook, *supra)*

The isolated nucleic acid compositions of this invention, such as RNA, cDNA, genomic DNA, or a hybrid thereof, can be obtained from biological sources using any number of cloning methodologies known to those of skill in the art. In some embodiments, oligonucleotide probes which selectively hybridize, under stringent conditions, to the polynucleotides of the present invention are used to identify the desired sequence in a cDNA or genomic DNA library. While isolation of RNA, and construction of cDNA and genomic libraries is well known to those of ordinary skill in the art. (See, e.g., Ausubel, *supra;* or Sambrook, *supra)*

A cDNA or genomic library can be screened using a probe based upon the sequence of a polynucleotide of the present invention, such as those disclosed herein. Probes may be used to hybridize with genomic DNA or cDNA sequences to isolate homologous genes in the same or different organisms. Those of skill in the art will appreciate that various degrees of stringency of hybridization can be employed in the assay; and either the hybridization or the wash medium can be stringent. As the conditions for hybridization become more stringent, there must be a greater degree of complementarity between the probe and the target for duplex formation to occur. The degree of stringency can be controlled by temperature, ionic strength, pH and the presence of a partially denaturing solvent such as formamide. For example, the stringency of hybridization is conveniently varied by changing the polarity of the reactant solution through, for example, manipulation of the concentration of formamide within the range of 0% to 50%. The degree of complementarity (sequence identity) required for detectable binding will vary in accordance with the stringency of the hybridization medium and/or wash medium. The degree of complementarity will optimally be 100%; however, it should be understood that minor sequence variations in the probes and primers may be compensated for by reducing the stringency of the hybridization and/or wash medium.

Methods of amplification of RNA or DNA are well known in the art and can be used according to the present invention without undue experimentation, based on the teaching and guidance presented herein.

Known methods of DNA or RNA amplification include, but are not limited to, polymerase chain reaction (PCR) and related amplification processes (see, e.g., U.S. Patent Nos. 4,683,195, 4,683,202, 4,800,159, 4,965,188, to Mullis, et al.; 4,795,699 and 4,921,794 to Tabor, et al; 5,142,033 to Innis; 5,122,464 to Wilson, et al.; 5,091,310 to Innis; 5,066,584 to Gyllensten, et al; 4,889,818 to Gelfand, et al; 4,994,370 to Silver, et al; 4,766,067 to Biswas; 4,656,134 to Ringold) and RNA mediated amplification which uses anti-sense RNA to the target sequence as a template for double-stranded DNA synthesis (U.S. Patent No. 5,130,238 to Malek, et al, with the tradename NASBA), the entire contents of which are herein incorporated by reference. (See, e.g., Ausubel, *supra;* or Sambrook, *supra)*

For instance, polymerase chain reaction (PCR) technology can be used to amplify the sequences of polynucleotides of the present invention and related genes directly from genomic DNA or cDNA libraries. PCR and other in vitro amplification methods may also be useful, for example, to clone nucleic acid sequences that code for proteins to be expressed, to make nucleic acids to use as probes for detecting the presence of the desired mRNA in samples, for nucleic acid sequencing, or for other purposes. Examples of techniques sufficient to direct persons of skill through in vitro amplification methods are found in Berger, Sambrook, and Ausubel, as well as Mullis, et al., U.S. Patent No. 4,683,202 (1987); and Innis, et al., PCR Protocols A Guide to Methods and Applications, Eds., Academic Press Inc., San Diego, CA (1990). Commercially available kits for genomic PCR amplification are known in the art. See, e.g., Advantage-GC Genomic PCR Kit (Clontech). The T4 gene 32 protein (Boehringer Mannheim) can be used to improve yield of long PCR products.

The isolated nucleic acids of the present invention can also be prepared by direct chemical synthesis by methods such as the phosphotriester method of Narang, et al., Meth. Enzymol. 68:90-99 (1979); the phosphodiester method of Brown, et al., Meth. Enzymol. 68:109-151 (1979); the diethylphosphoramidite method of Beaucage, et al., Tetra. Letts. 22:1859-1862 (1981); the solid phase phosphoramidite triester method described by Beaucage and Caruthers, Tetra. Letts. 22(20):1859-1862 (1981), e.g., using an automated synthesizer, e.g., as described in Needham-VanDevanter, et al., Nucleic Acids Res. 12:6159-6168 (1984); and the solid support method of U.S. Patent No. 4,458,066. Chemical synthesis generally produces a single-stranded oligonucleotide, which may be converted into double-stranded DNA by hybridization with a complementary sequence, or by polymerization with a DNA polymerase using the single strand as a template. One of skill in the art will recognize that while chemical synthesis of DNA can be limited to sequences of about 100 or more bases, longer sequences may be obtained by the ligation of shorter sequences.

The present invention further provides recombinant expression cassettes comprising a nucleic acid of the present invention. A nucleic acid sequence of the present invention, for example a cDNA or a genomic sequence encoding a full-length polypeptide of the present invention, can be used to construct a recombinant expression cassette which can be introduced into at least one desired host cell. A recombinant expression cassette will typically comprise a polynucleotide of the present invention operably linked to transcriptional initiation regulatory sequences that will direct the transcription of the polynucleotide in the intended host cell.

Both heterologous and non-heterologous (i.e., endogenous) promoters can be employed to direct expression of the nucleic acids of the present invention. These promoters can also be used, for example, in recombinant expression cassettes to drive expression of antisense nucleic acids to reduce, increase, or alter monkey FLINT content and/or composition in a desired tissue.

In some embodiments, isolated nucleic acids which serve as promoter or enhancer elements can be introduced in the appropriate position (generally upstream) of a non-heterologous form of a polynucleotide of the present invention so as to up or down regulate expression of a polynucleotide of the present invention. For example, endogenous promoters can be altered *in vivo* or *in vitro* by mutation, deletion and/or substitution.

A polynucleotide of the present invention can be expressed in either sense or anti-sense orientation as desired. It will be appreciated that control of gene expression in either sense or anti-sense orientation can have a direct impact on the observable characteristics.

Another method of suppression is sense suppression. Introduction of nucleic acid configured in the sense orientation has been shown to be an effective means by which to block the transcription of target genes.

A variety of cross-linking agents, alkylating agents and radical generating species as pendant groups on polynucleotides of the present invention can be used to bind, label, detect and/or cleave nucleic acids. Knorre, et al., Biochimie 67:785-789 (1985); Vlassov, et al., Nucleic Acids Res. 14:4065-4076 (1986); Iverson and Dervan, J. Am. Chem. Soc. 109:1241-1243 (1987); Meyer, et al., J. Am. Chem. Soc. 111:8517-8519 (1989); Lee, et al., Biochemistry 27:3197-3203 (1988); Home, et al., J. Am. Chem. Soc. 112:2435-2437 (1990); Webb and Matteucci, J. Am. Chem. Soc. 108:2764-2765 (1986); Nucleic Acids Res. 14:7661-7674 (1986); Feteritz, et al., J. Am. Chem. Soc. 113:4000 (1991). Various compounds to bind, detect, label, and/or cleave nucleic acids are known in the art. See, for example, U.S. Patent Nos. 5,543,507; 5,672,593; 5,484,908; 5,256,648; and 5,681941, each entirely incorporated herein by reference.

The present invention also relates to vectors that include isolated nucleic acid molecules of the present invention, host cells that are genetically engineered with the recombinant vectors, and the production of monkey FLINT polypeptides or fragments thereof by recombinant techniques, as is well known in the art. See, e.g., Sambrook, et al., 1989; Ausubel, et al., 1987-1998, each entirely incorporated herein by reference.

The polynucleotides can optionally be joined to a vector containing a selectable marker for propagation in a host. Generally, a plasmid vector is introduced in a precipitate, such as a calcium phosphate precipitate, or in a complex with a charged lipid. If the vector is a virus, it can be packaged in vitro using an appropriate packaging cell line and then transduced into host cells.

The DNA insert should be operatively linked to an appropriate promoter, such as the phage lambda PL promoter, the *E. coli lac, trp* and *tac* promoters, the SV40 early and late promoters and promoters of retroviral LTRs, or any other suitable promoter. Other suitable promoters will be known to the skilled artisan. The expression constructs will further contain sites for transcription initiation, termination and, in the transcribed region, a ribosome binding site for translation. The coding portion of the mature transcripts expressed by the constructs will preferably include a translation initiating at the beginning and a termination codon (e.g., UAA, UGA or UAG) appropriately positioned at the end of the mRNA to be translated, with UAA and UAG preferred for mammalian or eukaryotic cell expression.

Expression vectors will preferably include at least one selectable marker. Such markers include, e.g., dihydrofolate reductase, ampicillin (G418), or neomycin resistance for eukaryotic cell culture, and tetracycline or ampicillin resistance genes for culturing in *E.* coli and other bacteria or prokaryotics. Representative examples of appropriate hosts include, but are not limited to, bacterial cells, such as *E.* coli, Streptomyces and Salmonella typhimurium cells; fungal cells, such as yeast cells; insect cells such as Drosophila S2 and Spodoptera Sf9 cells; animal cells such as CHO, COS and Bowes melanoma cells; and plant cells. Appropriate culture mediums and conditions for the above-described host cells are known in the art. Vectors preferred for use in bacteria include pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRITS available from Pharmacia. Preferred eucaryotic vectors include pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of a vector construct into a host cell can be effected by calcium phosphate transfection, DEAE-dextran mediated transfection, cationic lipid-mediated transfection, electroporation, transduction, infection or other methods. Such methods are described in many standard laboratory manuals, such as Sambrook, supra, Chapters 1-4 and 16-18; Ausubel, supra, Chapters 1, 9, 13, 15, 16.

Polypeptide(s) of the present invention can be expressed in a modified form, such as a fusion protein, and can include not only secretion signals, but also additional heterologous functional regions. For instance, a region of additional amino acids, particularly charged amino acids, can be added to the N-terminus of a polypeptide to improve stability and persistence in the host cell, during purification, or during subsequent handling and storage. Also, peptide moieties can be added to a polypeptide to facilitate purification. Such regions can be removed prior to final preparation of a polypeptide. Such methods are described in many standard laboratory manuals, such as Sambrook, supra, Chapters 17.29-17.42 and 18.1-18.74; Ausubel, supra, Chapters 16, 17 and 18.

Using nucleic acids of the present invention, one may express a protein of the present invention in a recombinantly engineered cell, such as bacteria, yeast, insect, or mammalian cells. The cells produce the protein in a non-natural condition (e.g., in quantity, composition, location, and/or time), because they have been genetically altered through human intervention to do so.

It is expected that those of skill in the art are knowledgeable in the numerous expression systems available for expression of a nucleic acid encoding a protein of the present invention. No attempt to describe in detail the various methods known for the expression of proteins in prokaryotes or eukaryotes will be made.

In brief summary, the expression of isolated nucleic acids encoding a protein of the present invention will typically be achieved by operably linking, for example, the DNA or cDNA to a promoter (which is either constitutive or inducible), followed by incorporation into an expression vector. The vectors can be suitable for replication and integration in either prokaryotes or eukaryotes. Typical expression vectors contain transcription and translation terminators, initiation sequences and promoters useful for regulation of the expression of the DNA encoding a protein of the present invention. To obtain high level expression of a cloned gene, it is desirable to construct expression vectors which contain, at the minimum, a strong promoter to direct transcription, a ribosome binding site for translational initiation, and a transcription/translation terminator. One of skill would recognize that modifications can be made to a protein of the present invention without diminishing its biological activity. Some modifications may be made to facilitate the cloning, expression, or incorporation of the targeting molecule into a fusion protein. Such modifications are well known to those of skill in the art and include, for example, a methionine added at the amino terminus to provide an initiation site, or additional amino acids (e.g., poly His) placed on either terminus to create conveniently located restriction sites or termination codons or purification sequences.

Alternatively, nucleic acids of the present invention can be expressed in a host cell by turning on (by manipulation) in a host cell that contains endogenous DNA encoding a polypeptide of the present invention. Such methods are well known in the art, e.g., as described in US patent Nos. 5,580,734, 5,641,670, 5,733,746, and 5,733,761, entirely incorporated herein by reference.

Prokaryotic cells may be used as hosts for expression. Prokaryotes most frequently are represented by various strains of *E.* coli; however, other microbial strains may also be used. Commonly used prokaryotic control sequences which are defined herein to include promoters for transcription initiation, optionally with an operator, along with ribosome binding site sequences, include such commonly used promoters as the beta lactamase (penicillinase) and lactose (lac) promoter systems (Chang, et al., Nature 198:1056 (1977)), the tryptophan (trp) promoter system (Goeddel, et al., Nucleic Acids Res. 8:4057 (1980)) and the lambda derived P L promoter and N-gene ribosome binding site (Shimatake, et al., Nature 292:128 (1981)). The inclusion of selection markers in DNA vectors transfected in *E.* coli is also useful. Examples of such markers include genes specifying resistance to ampicillin, tetracycline, or chloramphenicol.

The vector is selected to allow introduction into the appropriate host cell. Bacterial vectors are typically of plasmid or phage origin. Appropriate bacterial cells are infected with phage vector particles or transfected with naked phage vector DNA. If a plasmid vector is used, the bacterial cells are transformed with the plasmid vector DNA. Expression systems for expressing a protein of the present invention are available using Bacillus sp. and Salmonella (Palva, et al., Gene 22:229-235 (1983); Mosbach, et al., Nature 302:543-545 (1983)).

A variety of eukaryotic expression systems such as yeast, insect cell lines, plant and mammalian cells, are known to those of skill in the art. As explained briefly below, a nucleic acid of the present invention can be expressed in these eukaryotic systems.

Synthesis of heterologous proteins in yeast is well known. F. Sherman, et al., Methods in Yeast Genetics, Cold Spring Harbor Laboratory (1982) is a well-recognized work describing the various methods available to produce the protein in yeast. Two widely utilized yeast for production of eukaryotic proteins are Saccharomyces cerevisiae and Pichia pastoris. Vectors, strains, and protocols for expression in Saccharomyces and Pichia are known in the art and available from commercial suppliers (e.g., Invitrogen). Suitable vectors usually have expression control sequences, such as promoters, including 3-phosphoglycerate kinase or alcohol oxidase, and an origin of replication, termination sequences and the like as desired.

A protein of the present invention, once expressed, can be isolated from yeast by lysing the cells and applying standard protein isolation techniques to the lysates. The monitoring of the purification process can be accomplished by using Western blot techniques or radioimmunoassay of other standard immunoassay techniques.

The sequences encoding proteins of the present invention can also be ligated to various expression vectors for use in transfecting cell cultures of, for instance, mammalian, insect, or plant origin. Illustrative of cell cultures useful for the production of the peptides are mammalian cells. Mammalian cell systems often will be in the form of monolayers of cells although mammalian cell suspensions may also be used. A number of suitable host cell lines capable of expressing intact proteins have been developed in the art, and include the HEK293, BHK21, and CHO cell lines. Expression vectors for these cells can include expression control sequences, such as an origin of replication, a promoter (e.g., the CMV promoter, a HSV tk promoter or pgk (phosphoglycerate kinase) promoter), an enhancer (Queen, et al., Immunol. Rev. 89:49 (1986)), and processing information sites, such as ribosome binding sites, RNA splice sites, polyadenylation sites (e.g., an SV40 large T Ag poly A addition site), and transcriptional terminator sequences. Other animal cells useful for production of proteins of the present invention are available, for instance, from the American Type Culture Collection Catalogue of Cell Lines and Hybridomas (7th edition, 1992).

Appropriate vectors for expressing proteins of the present invention in insect cells are usually derived from the SF9 baculovirus. Suitable insect cell lines include mosquito larvae, silkworm, armyworm, moth and Drosophila cell lines such as a Schneider cell line (See Schneider, J. Embryol. Exp. Morphol. 27:353-365 (1987).

As with yeast, when higher animal or plant host cells are employed, polyadenlyation or transcription terminator sequences are typically incorporated into the vector. An example of a terminator sequence is the polyadenlyation sequence from the bovine growth hormone gene. Sequences for accurate splicing of the transcript may also be included. An example of a splicing sequence is the VP1 intron from SV40 (Sprague, et al., J. Virol. 45:773-781 (1983)).

Additionally, gene sequences to control replication in the host cell may be incorporated into the vector such as those found in bovine papilloma virus type-vectors. M. Saveria-Campo, Bovine Papilloma Virus DNA, a Eukaryotic Cloning Vector in DNA Cloning Vol. II, a Practical Approach, D. M. Glover, Ed., IRL Press, Arlington, VA, pp. 213-238 (1985).

A FLINT polypeptide disclosed herein can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Polypeptides of the present invention include naturally purified products, products of chemical synthetic procedures, and products produced by recombinant techniques from a prokaryotic or eucaryotic host, including, for example, bacterial, yeast, higher plant, insect and mammalian cells. Depending upon the host employed in a recombinant production procedure, the polypeptides of the present invention can be glycosylated or can be non-glycosylated. In addition, polypeptides of the invention can also include an initial modified methionine residue, in some cases as a result of host-mediated processes. Such methods are described in many standard laboratory manuals, such as Sambrook, supra, Chapters 17.37-17.42; Ausubel, supra, Chapters 10, 12, 13, 16, 18 and 20.

The invention further provides an isolated monkey FLINT polypeptide (SEQ ID NO:4), variants thereof, and human FLINT polypeptide variants of SEQ ID NO:2.

FLINT is a novel TNF receptor molecule that encodes a 300 amino acid protein of which 29 residues comprise a leader sequence. A distinguishing feature of FLINT polypeptide is 20 cysteine residues at the amino terminal end of the molecule having a spacing pattern that is characteristic of the TNFR superfamily. FLINT shares approximately 30% sequence homology with TNFR at the amino terminal end while having no relatedness to the same superfamily at the carboxy end. The prototypical TNFR structure comprises four homologous structural and functional domains that are stabilized by disulfide bonds. Comparison of FLINT with the extracellular regions of TNFR I, Fas, and CD40 antigen from different species indicates that there are 9 structurally conserved disulfide bonds in FLINT spread over four domains, termed D1 through D4. Domain 1 (D1) is believed to contain two conserved disulfide bonds, with D2 having three conserved disulfide bonds, D3 having two conserved bonds, and D4 having two conserved bonds.

Sub-region D1 comprises the region approximately from residues 1 through 42 of SEQ ID NO:2 (or SEQ ID NO:4); D2 from approximately 43 through 85; D3 from approximately 86 through 122, and D4 from approximately residues 123 through 165 of SEQ ID NO:2 (or SEQ ID NO:4).

The present invention relates further to variants of human FLINT (SEQ ID NO:2) in which a single residue, alternatively one or more residues, of SEQ ID NO:2 is substituted conservatively by another amino acid.

The invention relates further to a human FLINT variant comprising a substitution(s) at one or more positions of SEQ ID NO:2 by a residue occurring at the same position in the monkey FLINT protein (SEQ ID NO:4). As the skilled artisan recognizes conservative amino acid changes typically do not alter the structure and/or function of a protein molecule nor, typically, do sequence differences that occur in closely related inter-species homologs. Thus, conservative amino acid changes to SEQ ID NO:2, based on substitution from the monkey sequence, and/or based on substitution by chemically similar amino acids, are expected to produce variants that retain FLINT activity. Therefore, the present invention relates further to human FLINT variants in which change(s) are made in the native sequence (SEQ ID NO:2) corresponding to substitution by residue(s) from the monkey FLINT sequence (SEQ ID NO:4). Single amino acid replacements, and/or multiple replacements including 2, 3, 4, 5 substitutions or greater are contemplated.

The invention relates further to substitutions in SEQ ID NO:2, wherein said substitutions occur within one or more of the sub-regions D1 through D4; alternatively in D1, alternatively in D2, alternatively in D3, alternatively in D4, alternatively in two or more of said sub-regions.

The proteins of the present invention or variants thereof can comprise any number of contiguous amino acid residues from a polypeptide of the present invention, wherein that number is selected from the group of integers consisting of from 90-100% of the number of contiguous residues in a full-length FLINT polypeptide. Optionally, this subsequence of contiguous amino acids is at least 50, 60, 70, 80, or 90 amino acids in length. Further, the number of such subsequences can be any integer selected from the group consisting of from 1 to 20, such as 2, 3, 4, or 5.

Generally, the polypeptides of the present invention will, when presented as an immunogen, elicit production of an antibody specifically reactive to a polypeptide of the present invention encoded by a polynucleotide of the present invention as described, supra. Exemplary polypeptides include those which are full-length, such as those disclosed herein. Further, the proteins of the present invention will not bind to antisera raised against a polypeptide of the present invention which has been fully immunosorbed with the same polypeptide. Immunoassays for determining binding are well known to those of skill in the art. A preferred immunoassay is a competitive immunoassay as discussed, infra. Thus, the proteins of the present invention can be employed as immunogens for constructing antibodies immunoreactive to a protein of the present invention for such exemplary utilities as immunoassays or protein purification techniques.

A FLINT polypeptide of the present invention can include one or more amino acid substitutions, deletions or additions, either from natural mutations or human manipulation, as specified herein.

Of course, the number of amino acid substitutions a skilled artisan would make depends on many factors, including those described above. Generally speaking, the number of amino acid substitutions, insertions or deletions for any given monkey FLINT polypeptide will not be more than 40, 30, 20, 10, 5, or 3, such as 1-30 or any range or value therein.

Amino acids in a monkey FLINT polypeptide of the present invention that are essential for function can be identified by methods known in the art, such as site-directed mutagenesis or alanine-scanning mutagenesis (Cunningham and Wells, Science 244:1081-1085 (1989)). The latter procedure introduces single alanine mutations at every residue in the molecule. The resulting mutant molecules are then routinely tested for biological activity.

Non-limiting mutants that can enhance or maintain at least one of the listed activities include, but are not limited to, any of the above polypeptides, further comprising at least one mutation corresponding to at least one substitution of SEQ ID NO:2.

In another aspect, the invention provides a peptide or polypeptide comprising an epitope-bearing portion of a polypeptide of the invention according to methods well known in the art. See, e.g., Colligan, et al,. ed., Current Protocols in Immunology, Greene Publishing, NY (1993-1998), Ausubel, *supra,* each entirely incorporated herein by reference.

The epitope of this polypeptide portion is an immunogenic or antigenic epitope of a polypeptide described herein. An "immunogenic epitope" can be defined as a part of a polypeptide that elicits an antibody response when the whole polypeptide is the immunogen. On the other hand, a region of a polypeptide molecule to which an antibody can bind is defined as an "antigenic epitope." The number of immunogenic epitopes of a polypeptide generally is less than the number of antigenic epitopes. See, for instance, Geysen, et al., Proc. Natl. Acad. Sci. USA 81:3998-4002 (1983) .

As to the selection of peptides or polypeptides bearing an antigenic epitope (i.e., that contain at least a portion of a region of a polypeptide molecule to which an antibody can bind), it is well known in the art that relatively short synthetic peptides that mimic part of a polypeptide sequence are routinely capable of eliciting an antiserum that reacts with the partially mimicked polypeptide. See, for instance, J. G. Sutcliffe, et al., "Antibodies that react with preidentified sites on polypeptides," Science 219:660-666 (1983).

Antigenic epitope-bearing peptides and polypeptides of the invention are useful to raise antibodies, including monoclonal antibodies, or screen antibodies, including fragments or single chain antibodies, that bind specifically to a polypeptide of the invention. See, for instance, Wilson, et al., Cell 37:767-778 (1984) at 777. Antigenic epitope-bearing peptides and polypeptides of the invention preferably contain a sequence of at least five, more preferably at least nine, and most preferably between at least about 15 to about 30 amino acids contained within the amino acid sequence of a polypeptide of the invention.

The epitope-bearing peptides and polypeptides of the invention can be produced by any conventional means. R. A. Houghten, "General method for the rapid solid-phase synthesis of large numbers of peptides: specificity of antigen-antibody interaction at the level of individual amino acids," Proc. Natl. Acad. Sci. USA 82:5131-5135 (1985). This "Simultaneous Multiple Peptide Synthesis (SMPS)" process is further described in U.S. Patent No. 4,631,211 to Houghten, et al. (1986).

As one of skill in the art will appreciate, monkey FLINT polypeptides of the present invention and the epitope-bearing fragments thereof described above can be combined with parts of the constant domain of immunoglobulins (IgG), resulting in chimeric polypeptides. These fusion proteins facilitate purification and show an increased half-life in vivo. This has been shown, e.g., for chimeric proteins consisting of the first two domains of the human CD4-polypeptide and various domains of the constant regions of the heavy or light chains of mammalian immunoglobulins (EPA 394,827; Traunecker, et al., Nature 331:84-86 (1988)). Fusion proteins that have a disulfide-linked dimeric structure due to the IgG part can also be more efficient in binding and neutralizing other molecules than the monomeric monkey FLINT polypeptide or polypeptide fragment alone (Fountoulakis, et al., J. Biochem. 270:3958-3964 (1995)).

The polypeptides of this invention and fragments thereof may be used in the production of antibodies. The term "antibody" as used herein describes antibodies, fragments of antibodies (such as, but not limited, to Fab, Fab', Fab2', and Fv fragments), and modified versions thereof, as well known in the art (e.g., chimeric, humanized, recombinant, veneered, resurfaced or CDR-grafted) such antibodies are capable of binding antigens of a similar nature as the parent antibody molecule from which they are derived. The instant invention also encompasses single chain polypeptide binding molecules.

The production of antibodies, both monoclonal and polyclonal, in animals is well known in the art. See, e.g., Colligan, supra, entirely incorporated herein by reference.

Single chain antibodies and libraries thereof are yet another variety of genetically engineered antibody technology that is well known in the art. (See, e.g., R. E. Bird, et al., Science 242:423-426 (1988); PCT Publication Nos. WO 88/01649, WO 90/14430, and WO 91/10737. Single chain antibody technology involves covalently joining the binding regions of heavy and light chains to generate a single polypeptide chain. The binding specificity of the intact antibody molecule is thereby reproduced on a single polypeptide chain.

Antibodies included in this invention are useful in diagnostics, therapeutics or in diagnostic/therapeutic combinations.

The polypeptides of this invention or suitable fragments thereof can be used to generate polyclonal or monoclonal antibodies, and various inter-species hybrids, or humanized antibodies, or antibody fragments, or single-chain antibodies. The techniques for producing antibodies are well known to skilled artisans. (See, e.g., Colligan supra; Monoclonal Antibodies: Principles & Applications, Ed. J. R. Birch & E. S. Lennox, Wiley-Liss (1995).

A polypeptide used as an immunogen may be modified or administered in an adjuvant, by subcutaneous or intraperitoneal injection into, for example, a mouse or a rabbit. For the production of monoclonal antibodies, spleen cells from immunized animals are removed, fused with myeloma or other suitable known cells, and allowed to become monoclonal antibody producing hybridoma cells in the manner known to the skilled artisan. Hybridomas that secrete a desired antibody molecule can be screened by a variety of well known methods, for example ELISA assay, Western blot analysis, or radioimmunoassay (Lutz, et al. Exp. Cell Res. 175:109-124 (1988); Monoclonal Antibodies: Principles & Applications, Ed. J. R. Birch & E. S. Lennox, Wiley-Liss (1995); Colligan, supra).

For some applications labeled antibodies are desirable. Procedures for labeling antibody molecules are widely known, including for example, the use of radioisotopes, affinity labels, such as biotin or avidin, enzymatic labels, for example horseradish peroxidase, and fluorescent labels, such as FITC or rhodamine (See, e.g., Colligan, supra).

Labeled antibodies are useful for a variety of diagnostic applications. In one embodiment the present invention relates to the use of labeled antibodies to detect the presence of a monkey FLINT polypeptide. Alternatively, the antibodies could be used in a screen to identify potential modulators of a monkey FLINT polypeptide. For example, in a competitive displacement assay, the antibody or compound to be tested is labeled by any suitable method. Competitive displacement of an antibody from an antibody-antigen complex by a test compound such that a test compound-antigen complex is formed provides a method for identifying compounds that bind HPLFP.

The present invention is also directed to a transgenic non-human eukaryotic animal (preferably a rodent, such as a mouse) the germ cells and somatic cells of which contain nucleic acid genomic DNA according to the present invention which codes for at least one monkey FLINT polypeptide. At least one monkey FLINT nucleic acid can be introduced into the animal to be made transgenic, or an ancestor of the animal, at an embryonic stage, preferably the 1-1000 cell or oocyte, stage, and preferably not later than about the 64-cell stage. The term "transgene," as used herein, means a gene which is incorporated into the genome of the animal and is expressed in the animal, resulting in the presence of at least one monkey FLINT polypeptide in the transgenic animal.

There are several means by which such a monkey FLINT nucleic acid can be introduced into a cell or genome of the animal embryo so as to be chromosomally incorporated and expressed according to known methods.

Chimeric non-human mammals in which fewer than all of the somatic and germ cells contain the a monkey FLINT polypeptide nucleic acid of the present invention, such as animals produced when fewer than all of the cells of the morula are transfected in the process of producing the transgenic animal, are also intended to be within the scope of the present invention.

Chimeric non-human mammals having human cells or tissue engrafted therein are also encompassed by the present invention, which may be used for testing expression of at least one monkey FLINT polypeptide in human tissue and/or for testing the effectiveness of therapeutic and/or diagnostic agents associated with delivery vectors which preferentially bind to a monkey FLINT polypeptide of the present invention. Methods for providing chimeric non-human mammals are provided, e.g., in U.S. Serial Nos. 07/508,225, 07/518,748, 07/529,217, 07/562,746, 07/596,518, 07/574,748, 07/575,962, 07/207,273, 07/241,590 and 07/137,173, which are entirely incorporated herein by reference, for their description of how to engraft human cells or tissue into non-human mammals.

The techniques described in Leder, U.S. Patent No. 4,736,866 (hereby entirely incorporated by reference) for producing transgenic non-human mammals may be used for the production of a transgenic non-human mammal of the present invention. The various techniques described in U.S. patent Nos. 5,454,807, 5,073,490, 5,347,075 and 4,736,866, the entire contents of which are hereby incorporated by reference, may also be used.

Animals carrying at least one monkey FLINT polypeptide and/or nucleic acid can be used to test compounds or other treatment modalities which may prevent, suppress or cure a pathology relating to at least one monkey FLINT polypeptide or monkey FLINT nucleic acid. Such transgenic animals will also serve as a model for testing of diagnostic methods for the same diseases. Transgenic animals according to the present invention can also be used as a source of cells for cell culture.

The clinical utility for FLINT and FLINT variants is expected to be substantial. Many diseases and/or conditions involving FasL/Fas are potentially amenable to therapy with FLINT and variants thereof. Examples of suitable diseases and/or conditions include but are not limited to:

Inflammatory/autoimmune diseases - Rheumatoid arthritis, inflammatory bowel disease, graft-versus-host disease, insulin-dependent diabetes, SIRS/sepsis/MODS/ARDS, pancreatitis, psoriasis, multiple sclerosis, Hashimoto's thyroiditis, Grave's disease, transplant rejection, SLE, autoimmune gastritis, fibrosing lung disease.

Infectious diseases - HIV-induced lymphopenia, fulminant viral hepatitis B/C, chronic hepatitis/cirrhosis, H. pylori-associated ulceration.

Ischemia/Re-perfusion conditions - Acute coronary syndrome, acute myocardial infarction, congestive heart failure, atherosclerosis, acute cerebral ischemia/infarction, brain/spinal cord trauma, organ preservation during transplant

Other - Cytoprotection during cancer treatment, adjuvant to chemotherapy, Alzheimer's, chronic glomerulonephritis, osteoporosis, TTP/HUS, aplastic anemia, myelodysplasia. Also of interest are treatment and prevention of acute lung injury (ALI)/acute respiratory distress syndrome (ARDS); Ulcerative colitis; and Crohn's disease.

Suitable therapeutic applications for FLINT and variants thereof also include prevention of ALI/ARDS in high risk patients (e.g., those with sepsis or hemorrhagic shock). As is the case for ALI/ARDS, there are data from animals and human studies suggesting an important role of FasL/Fas in the pathogeneses of ulcerative colitis and organ damage during transplantation.

Runaway apoptosis is an example of a condition caused by excessive activation of the FAS/FAS Ligand signal transduction pathway that can be treated with FLINT polypeptides of the present invention (See Kondo et *al.,* Nature Medicine 3(4):409-413 (1997) and Galle *et al., J. Exp. Med.* 182:1223-1230 (1995), the entire teachings of which are incorporated herein by reference). Runaway apoptosis can cause pathological conditions such as organ failure in the subject. Diseases associated with runaway apoptosis include, but are not limited to, acute liver failure (e.g., liver failure associated with viral infections affecting the liver, bacterial infections affecting the liver, hepatitis, hepatocellular injury and/or other conditions where hepatocytes undergo massive apoptosis or destruction), kidney failure and failure of pancreatic function.

The FLINT polypeptides and human FLINT variants of the present invention are generally therapeutically useful for diseases which can be treated with human FLINT. (See U.S. Provisional Application Serial No. 60/112,577; U.S. Application Serial No. 09/280,567; and Miwa et *al., Nature Medicine* 4:1287 (1998), the entire teachings of which are incorporated herein by reference). One example is inflammation caused by FAS Ligand induced neutrophil activation. Inflammatory disease associated with neutrophil activation include sepsis, ARDS, SIRS and MODS.

Other diseases for which the FLINT polypeptides of the present invention are useful include Rheumatoid arthritis (Elliott *et al., Lancet* 344:1105-10 (1994)), acute lung injury, ARDS, HIV (Dockrell *et al., J. Clin. Invest.* 101:2394-2405 (1998)), Ischemia (Sakurai et al. 1998 Brain Res 797:23-28), Brain trauma/injury (Ertel et al. 1997 J Neuroimmunol 80:93-6), chronic renal failure (Schelling et al. 1998 Lab Invest 78:813-824), Graft-vs-Host Disease (GVHD) (Hattori et al. 1998 Blood 11:4051-4055), Cutaneous inflammation (Orteu at al. 1998 J Immunol 161:1619-1629), Vascular leak syndrome (Rafi et al. 1998 J Immunol 161:3077-3086), Helicobacter pylori infection (Rudi et al. 1998 J Clin Invest 102:1506-1514), Goiter (Tamura et al. 1998 Endocrinology 139:3646-3653), Atherosclerosis (Sata and Walsh, 1998 J Clin Invest 102:1682-1689), IDDM (Itoh et al. 1997 J Exp Med 186:613-618), Osteoporosis (Jilka et al. 1998 J Bone Min Res 13:793-802), Crohn's Disease (van Dullemen et al. 1995 Gastroenterology 109:129-35), organ preservation and transplant (graft) rejection (Lau et al. 1996 Science 273:109-112), Sepsis (Faist and Kim. 1998 New Horizons 6:S97-102), Pancreatitis (Neoptolemos et al. 1998 Gut 42:886-91), Cancer (melanoma, colon and esophageal) (Bennett et al. 1998 J Immunol 160:5669-5675), Autoimmune disease (IBD, psoriasis, Down's Syndrome (Seidi et *al., Neuroscience Lett. 260:9* (1999) and multiple sclerosis (D'Souza et al. 1996 J Exp Med 184:2361-70).

Co-pending U.S. Patent Application, Serial No. 09/280567, entitled "Therapeutic Applications of mFLINT Polypeptides," filed March 30, 1999 discloses other diseases which can be treated with FLINT and variants. Examples include Alzheimer's Disease; End-stage renal disease (ESRD); mononulceosis; EBV; Herpes; ulcerative colitis; antibody dependent sytotoxicity; hemolytic and hypercoagulation disorders such as vascular bleeds, DIC (disseminated intervascular coagulation), eclampsia, HELLP (preeclampsia complicated by thrombocytopenia, hemolysis and disturbed liver function), HITS (heparin induced thromobcytopenia), HUS (hemolytic uremic syndrome), and preeclampsia; hematopoeitic disorders such as aplastic anemia, thrombocytopenia (TTP) and myelodysplasia; and hemolytic fever caused, for example, by E.bola.

The ability of FLINT and variants to retard apoptosis under ischemic conditions is useful also in preserving organs and tissues harvested for transplantation. Ischemic conditions include, but are not limited to, neuronal ischemia, limb crush injuries, spinal cord injuries, myocardial infarct including acute, sub-acute and chronic sequelae and related clinical syndromes, congestive heart failure. Also, innocent bystander tissues which are damaged during chemotherapy, radiation therapy, toxic drugs, trauma, surgery and other stresses can be treated with FLINT or FLINT variant. One example of such a disease is mucositis which can be a life-threatening side-effect of cancer treatment.

In another embodiment, the invention relates to the use of FLINT and variants to prevent and/or inhibit apoptosis in organs harvested from donors for transplantation. In this aspect of the invention, FLINT and/or a variant may be administered to the organ donor prior to harvesting the organ. After harvesting the organ, FLINT or variant is added to a suitable medium for transport/storage of the organ. Alternatively, the harvested organ is perfused with a medium containing FLINT or variant prior to transplantation into a recipient. Suitable media for perfusion are known, for example, the medium disclosed in EP 0356367 A2, herein incorporated by reference. The method may also include treating the transplant recipient with FLINT or FLINT variant prior to and/or after the transplant surgery.

For example, an organ donor is pre-treated with an effective amount of FLINT or variant prior to organ harvesting. Alternatively, or conjunctively, the harvested organ may be perfused or bathed in a solution containing FLINT analog. The method may be employed, for example, with kidney, heart, lung and other organs and tissues.

Acute lung injury (ALI) and acute respiratory distress syndrome (ARDS) represent disease entities that differ only in the severity of the hypoxemia present at diagnosis. A widely accepted parameter for diagnosis is the PaO2 to FiO2 ratio, according to which ARDS patients manifest a ratio of less than or equal to 200 mm Hg, whereas ALI patients exhibit values of less than or equal to 300 mm Hg. ARDS represents a more severe form of ALI. Numerous mediators are likely to contribute to the pathogenesis of ARDS/ALI with neutrophils playing a prominent role. While multiple precipitating factors are probable in the development of ARDS, both direct and indirect, the major cause appears to be sepsis and the systemic inflammatory response syndrome, accounting for approximately 40% of cases. Mortality in ARDS is high approximating 40%, with most deaths occurring within the first 2 to 3 weeks. There is no currently available, approved pharmacologic therapy for ARDS and treatment at present is limited to aggressive supportive care.

There is evidence that ARDS may be mediated by soluble FasL/Fas interaction in humans (Matute-Bello et al., J. Immunol. 163, 2217-2225, 1999). FLINT analog, by binding to FasL, could inhibit FasL-mediated apoptosis of pneumocytes and/or endothelial cells, thus inhibiting or preventing the progression from acute inflammatory insult to ALI, and from ALI to ARDS.

In one embodiment the present invention relates to the use of FLINT or variant to inhibit and/or treat ALI and/or ARDS comprising the administration of a therapeutically effective amount of FLINT or variant to a person in need thereof.

A "subject" is a mammal in need of treatment, preferably a human, but can also be an animal in need of veterinary treatment, e.g., domestic animals (e.g., dogs, cats, and the like), farm animals (e.g., cows, sheep, pigs, horses, and the like) and laboratory animals (e.g., rats, mice, guinea pigs, and the like).

An "effective amount" of a FLINT or variant is an amount which results in a sufficient inhibition of one or more processes mediated by the binding of FAS to FAS Ligand or LIGHT to LTβR and/or TR2/HVEM so as to achieve a desired therapeutic or prophylactic effect in a subject with a disease or condition associated with aberrant FAS/FAS Ligand binding and/or LIGHT mediated binding. One example of such a process is runaway apoptosis. Alternatively, an "effective amount" of a FLINT or variant is a quantity sufficient to achieve a desired therapeutic and/or prophylactic effect in a subject with inflammation caused by FAS Ligand induced neutrophil activation or any of the other aforementioned diseases associated with aberrant FAS Ligand activity.

A "desired therapeutic and/or prophylactic effect" in a subject with a disease or condition includes the amelioration of symptoms, or delay in onset of symptoms, associated with such disease. Alternatively, a "desired therapeutic and/or prophylactic effect" includes an increased survival rate or increased longevity for the subject with the disease.

The amount of FLINT administered to the individual will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors.

As a general proposition, the total pharmaceutically effective amount of the FLINT or variant of the present invention administered parenterally per dose will be in the range of about 1 µg/kg/day to 10 mg/kg/day of patient body weight, particularly 2 mg/kg/day to 8 mg/kg/day, more particularly 2 mg/kg/day to 4 mg/kg/day, even more particularly 2.2 mg/kg/day to 3.3 mg/kg/day, and finally 2.5 mg/kg/day, although, as noted above, this will be subject to therapeutic discretion. More preferably, this dose is at least 0.01 mg/kg/day. If given continuously, the FLINT or variants of the present invention are typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the FLINT polypeptides of the present invention may be administered orally, rectally, intracranially, parenterally, intracisternally, intravaginally, intraperitoneally, topically (as by powders, ointments, drops or transdermal patch), transdermally, intrathecally, bucally, or as an oral or nasal spray. By "pharmaceutically acceptable carrier" is meant a non-toxic solid, semisolid or liquid filler, diluent, encapsulating material or formulation auxiliary of any type. The term "parenteral" as used herein includes, but is not limited to, modes of administration which include intravenous, intramuscular, intraperitoneal, intrasternal, subcutaneous and intraarticular injection, infusion and implants comprising FLINT or variants.

The FLINT or variants of the present invention are also suitably administered by sustained-release systems. Suitable examples of sustained-release compositions include semi-permeable polymer matrices in the form of shaped articles, e.g., films, or microcapsules. Sustained-release matrices include polylactides (U.S. Pat. No. 3,773.919, EP 58,481), copolymers of L-glutamic acid and gamma-ethyl-L-glutamate (Sidman, U. et al., *Biopolymers* 22:547-556 (1983)), poly (2-hydroxyethyl methacrylate) (R.Langer et al., *J. Biomed. Mater. Res.* 15:167-277 (1981), and R. Langer, *Chem. Tech.* 12:98-105 (1982)), ethylene vinyl acetate (R. Langer et al., Id.) or poly-D-(-)-3-hydroxybutyric acid (EP 133,988). Other sustained-release compositions also include liposomally entrapped FLINT analog. Such liposomes are prepared by methods known per se: DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EDP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal TNFR polypeptide therapy.

For parenteral administration, in one embodiment, the FLINT or variants of the present invention are formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the FLINT or variants of the present invention uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The FLINT or variants of the present invention are typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of salts of the FLINT analogs of the present invention.

Polypeptides to be used for therapeutic administration must be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

FLINT compositions ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution of one of the FLINT analogs of the present invention, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the FLINT or variants of the present invention may be employed in conjunction with other therapeutical compounds.

Having generally described the invention, the same will be more readily understood by reference to the following examples, which are provided by way of illustration and are not intended as limiting.

### Example 1

### Expression and Purification of a Monkey FLINT Polypeptide in E. coli

The bacterial expression vector pQE60 is used for bacterial expression in this example. (QIAGEN, Inc., Chatsworth, CA). pQE60 encodes ampicillin antibiotic resistance ("Ampr") and contains a bacterial origin of replication ("ori"), an IPTG inducible promoter, a ribosome binding site ("RBS"), six codons encoding histidine residues that allow affinity purification using nickel-nitrilo-triacetic acid ("Ni-NTA") affinity resin sold by QIAGEN, Inc., and suitable single restriction enzyme cleavage sites. These elements are arranged such that a DNA fragment encoding a polypeptide can be inserted in such a way as to produce that polypeptide with the six His residues (i.e., a "6 X His tag") covalently linked to the carboxyl terminus of that polypeptide. However, a polypeptide coding sequence can optionally be inserted such that translation of the six His codons is prevented and, therefore, a polypeptide is produced with no 6 X His tag.

The nucleic acid sequence encoding the desired portion of a monkey FLINT polypeptide lacking the hydrophobic leader sequence is amplified from the deposited cDNA clone using PCR oligonucleotide primers (based on the sequences presented, e.g., as presented in at least one of SEQ ID NO:1, ), which anneal to the amino terminal encoding DNA sequences of the desired portion of a monkey FLINT polypeptide and to sequences in the deposited construct 3' to the cDNA coding sequence. Additional nucleotides containing restriction sites to facilitate cloning in the pQE60 vector are added to the 5' and 3' sequences, respectively.

For cloning a monkey FLINT polypeptide, the 5' and 3' primers have nucleotides corresponding or complementary to a portion of the coding sequence of a monkey FLINT, e.g., as presented in at least one of SEQ ID NO:1, according to known method steps. One of ordinary skill in the art would appreciate, of course, that the point in a polypeptide coding sequence where the 5' primer begins can be varied to amplify a desired portion of the complete polypeptide shorter or longer than the mature form.

The amplified monkey FLINT nucleic acid fragments and the vector pQE60 are digested with appropriate restriction enzymes and the digested DNAs are then ligated together. Insertion of the monkey FLINT DNA into the restricted pQE60 vector places a monkey FLINT polypeptide coding region including its associated stop codon downstream from the IPTG-inducible promoter and in-frame with an initiating AUG codon. The associated stop codon prevents translation of the six histidine codons downstream of the insertion point.

The ligation mixture is transformed into competent E. coli cells using standard procedures such as those described in Sambrook, et al., 1989; Ausubel, 1987-1998. *E.* coli strain M15/rep4, containing multiple copies of the plasmid pREP4, which expresses the lac repressor and confers kanamycin resistance, is used in carrying out the illustrative example described herein. This strain, which is only one of many that are suitable for expressing monkey FLINT polypeptide, is available commercially from QIAGEN, Inc. Transformants are identified by their ability to grow on LB plates in the presence of ampicillin and kanamycin. Plasmid DNA is isolated from resistant colonies and the identity of the cloned DNA confirmed by restriction analysis, PCR and DNA sequencing.

Clones containing the desired constructs are grown overnight ("O/N") in liquid culture in LB media supplemented with both ampicillin (100 µg/ml) and kanamycin (25 µg/ml). The O/N culture is used to inoculate a large culture, at a dilution of approximately 1:25 to 1:250. The cells are grown to an optical density at 600 nm ("OD600") of between 0.4 and 0.6. Isopropyl-b-D-thiogalactopyranoside ("IPTG") is then added to a final concentration of 1 mM to induce transcription from the lac repressor sensitive promoter, by inactivating the lacI repressor. Cells subsequently are incubated further for 3 to 4 hours. Cells then are harvested by centrifugation.

The cells are then stirred for 3-4 hours at 4°C in 6M guanidine-HCl, pH8. The cell debris is removed by centrifugation, and the supernatant containing the monkey FLINT is dialyzed against 50 mM Na-acetate buffer pH6, supplemented with 200 mM NaCl. Alternatively, a polypeptide can be successfully refolded by dialyzing it against 500 mM NaCl, 20% glycerol, 25 mM Tris/HCl pH7.4, containing protease inhibitors.

If insoluble protein is generated, the protein is made soluble according to known method steps. After renaturation the polypeptide is purified by ion exchange, hydrophobic interaction and size exclusion chromatography. Alternatively, an affinity chromatography step such as an antibody column is used to obtain pure monkey FLINT polypeptide. The purified polypeptide is stored at 4°C or frozen at -40°C to -120°C.

### Example 2

### Cloning and Expression of a monkey FLINT Polypeptide in a Baculovirus Expression System

In this illustrative example, the plasmid shuttle vector pA2 GP is used to insert the cloned DNA encoding the mature polypeptide into a baculovirus to express a monkey FLINT polypeptide, using a baculovirus leader and standard methods as described in Summers, et al., A Manual of Methods for Baculovirus Vectors and Insect Cell Culture Procedures, Texas Agricultural Experimental Station Bulletin No. 1555 (1987). This expression vector contains the strong polyhedrin promoter of the Autographa californica nuclear polyhedrosis virus (AcMNPV) followed by the secretory signal peptide (leader) of the baculovirus gp67 polypeptide and convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from E. coli under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate viable virus that expresses the cloned polynucleotide.

Other baculovirus vectors are used in place of the vector above, such as pAc373, pVL941 and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow, et al., Virology 170:31-39.

The cDNA sequence encoding the mature monkey FLINT polypeptide in the deposited or other clone, lacking the AUG initiation codon and the naturally associated nucleotide binding site, is amplified using PCR oligonucleotide primers corresponding to the 5' and 3' sequences of the gene. Non-limiting examples include 5' and 3' primers having nucleotides corresponding or complementary to a portion of the coding sequence of a monkey FLINT polypeptide, e.g., as presented in at least one of SEQ ID NO:1, according to known method steps.

The amplified fragment is isolated from a 1% agarose gel using a commercially available kit (e.g., "Geneclean," BIO 101 Inc., La Jolla, CA). The fragment then is then digested with the appropriate restriction enzyme and again is purified on a 1% agarose gel. This fragment is designated herein "F1".

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, CA). This vector DNA is designated herein "V1".

Fragment F1 and the dephosphorylated plasmid V1 are ligated together with T4 DNA ligase. E. coli HB101 or other suitable *E.* coli hosts such as XL-1 Blue (Stratagene Cloning Systems, La Jolla, CA) cells are transformed with the ligation mixture and spread on culture plates. Bacteria are identified that contain the plasmid with the human monkey FLINT gene using the PCR method, in which one of the primers that is used to amplify the gene and the second primer is from well within the vector so that only those bacterial colonies containing the monkey FLINT gene fragment will show amplification of the DNA. The sequence of the cloned fragment is confirmed by DNA sequencing. This plasmid is designated herein pBac monkey FLINT .

Five µg of the plasmid pBacmonkey FLINT is cotransfected with 1.0 µg of a commercially available linearized baculovirus DNA ("BaculoGold™ baculovirus DNA", Pharmingen, San Diego, CA), using the lipofection method described by Felgner, et al., Proc. Natl. Acad. Sci. USA 84:7413-7417 (1987). 1 µg of BaculoGold™ virus DNA and 5 µ g of the plasmid pBac monkey FLINT are mixed in a sterile well of a microtiter plate containing 50 µl of serum-free Grace's medium (Life Technologies, Inc., Rockville, MD). Afterwards, 10 µl Lipofectin plus 90 µl Grace's medium are added, mixed and incubated for 15 minutes at room temperature. Then the transfection mixture is added dropwise to Sf9 insect cells (ATCC CRL 1711) seeded in a 35 mm tissue culture plate with 1 ml Grace's medium without serum.

The plate is rocked back and forth to mix the newly added solution. The plate is then incubated for 5 hours at 27°C. After 5 hours the transfection solution is removed from the plate and 1 ml of Grace's insect medium supplemented with 10% fetal calf serum is added. The plate is put back into an incubator and cultivation is continued at 27°C for four days.

After four days the supernatant is collected and a plaque assay is performed, according to known methods. An agarose gel with "Blue Gal" (Life Technologies, Inc., Rockville, MD) is used to allow easy identification and isolation of gal-expressing clones, which produce blue-stained plaques. (A detailed description of a "plaque assay" of this type can also be found in the user's guide for insect cell culture and baculovirology distributed by Life Technologies, Inc., Rockville, MD, page 9-10). After appropriate incubation, blue stained plaques are picked with a micropipettor tip (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4°C. The recombinant virus is called V-monkey FLINT.

To verify the expression of the monkey FLINT gene, Sf9 cells are grown in Grace's medium supplemented with 10% heat-inactivated FBS. The cells are infected with the recombinant baculovirus V-monkey FLINT at a multiplicity of infection ("MOI") of about 2. Six hours later the medium is removed and is replaced with SF900 II medium minus methionine and cysteine (available, e.g., from Life Technologies, Inc., Rockville, MD). If radiolabeled polypeptides are desired, 42 hours later, 5 mCi of 35S-methionine and 5 mCi 35S-cysteine (available from Amersham) are added. The cells are further incubated for 16 hours and then they are harvested by centrifugation. The polypeptides in the supernatant as well as the intracellular polypeptides are analyzed by SDS-PAGE followed by autoradiography (if radiolabeled). Microsequencing of the amino acid sequence of the amino terminus of purified polypeptide can be used to determine the amino terminal sequence of the mature polypeptide and thus the cleavage point and length of the secretory signal peptide.

### Example 3

### Cloning and Expression of monkey FLINT in Mammalian Cells

A typical mammalian expression vector contains at least one promoter element, which mediates the initiation of transcription of mRNA, the polypeptide coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as pIRESlneo, pRetro-Off, pRetro-On, PLXSN, or pLNCX (Clonetech Labs, Palo Alto, CA), pcDNA3.1 (+/-), pcDNA/Zeo (+/-) or pcDNA3.1/Hygro (+/-) (Invitrogen), PSVL and PMSG (Pharmacia, Uppsala, Sweden), pRSVcat (ATCC 37152), pSV2dhfr (ATCC 37146) and pBC12MI (ATCC 67109). Mammalian host cells that could be used include human Hela 293, H9 and Jurkat cells, mouse NIH3T3 and C127 cells, Cos 1, Cos 7 and CV 1, quail QC1-3 cells, mouse L cells and Chinese hamster ovary (CHO) cells.

Alternatively, the gene can be expressed in stable cell lines that contain the gene integrated into a chromosome. The co-transfection with a selectable marker such as dhfr, gpt, neomycin, or hygromycin allows the identification and isolation of the transfected cells.

The transfected gene can also be amplified to express large amounts of the encoded polypeptide. The DHFR (dihydrofolate reductase) marker is useful to develop cell lines that carry several hundred or even several thousand copies of the gene of interest. Another useful selection marker is the enzyme glutamine synthase (GS) (Murphy, et al., Biochem. J. 227:277-279 (1991); Bebbington, et al., Bio/Technology 10:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of polypeptides.

The expression vectors pC1 and pC4 contain the strong promoter (LTR) of the Rous Sarcoma Virus (Cullen, et al., Molec. Cell. Biol. 5:438-447 (1985)) plus a fragment of the CMV-enhancer (Boshart, et al., Cell 41:521-530 (1985)). Multiple cloning sites, e.g., with the restriction enzyme cleavage sites BamHI, XbaI and Asp718, facilitate the cloning of the gene of interest. The vectors contain in addition the 3' intron, the polyadenylation and termination signal of the rat preproinsulin gene.

### Example 4

### Cloning and Expression in COS Cells

The expression plasmid, pmonkey FLINT HA, is made by cloning a cDNA encoding monkey FLINT into the expression vector pcDNAI/Amp or pcDNAIII (which can be obtained from Invitrogen, Inc.).

The expression vector pcDNAI/amp contains: (1) an E. coli origin of replication effective for propagation in E. coli and other prokaryotic cells; (2) an ampicillin resistance gene for selection of plasmid-containing prokaryotic cells; (3) an SV40 origin of replication for propagation in eucaryotic cells; (4) a CMV promoter, a polylinker, an SV40 intron; (5) several codons encoding a hemagglutinin fragment (i.e., an "HA" tag to facilitate purification) or HIS tag (see, e.g, Ausubel, supra) followed by a termination codon and polyadenylation signal arranged so that a cDNA can be conveniently placed under expression control of the CMV promoter and operably linked to the SV40 intron and the polyadenylation signal by means of restriction sites in the polylinker. The HA tag corresponds to an epitope derived from the influenza hemagglutinin polypeptide described by Wilson, et al., Cell 37:767-778 (1984). The fusion of the HA tag to the target polypeptide allows easy detection and recovery of the recombinant polypeptide with an antibody that recognizes the HA epitope. pcDNAIII contains, in addition, the selectable neomycin marker.

A DNA fragment encoding the monkey FLINT is cloned into the polylinker region of the vector so that recombinant polypeptide expression is directed by the CMV promoter. The plasmid construction strategy is as follows. The monkey FLINT cDNA of the deposited clone is amplified using primers that contain convenient restriction sites, much as described above for construction of vectors for expression of monkey FLINT in E. coli. Non-limiting examples of suitable primers include those based on the coding sequences presented in at least one of SEQ ID NO:1, as they encode monkey FLINT polypeptides as described herein.

The PCR amplified DNA fragment and the vector, pcDNAI/Amp, are digested with suitable restriction enzyme(s) and then ligated. The ligation mixture is transformed into E. coli strain SURE (available from Stratagene Cloning Systems, 11099 North Torrey Pines Road, La Jolla, CA 92037), and the transformed culture is plated on ampicillin media plates which then are incubated to allow growth of ampicillin resistant colonies. Plasmid DNA is isolated from resistant colonies and examined by restriction analysis or other means for the presence of the monkey FLINT-encoding fragment.

For expression of recombinant monkey FLINT, COS cells are transfected with an expression vector, as described above, using DEAE-DEXTRAN, as described, for instance, in Sambrook, et al., Molecular Cloning: a Laboratory Manual, Cold Spring Laboratory Press, Cold Spring Harbor, New York (1989). Cells are incubated under conditions for expression of monkey FLINT by the vector.

Expression of the monkey FLINT-HA fusion polypeptide is detected by radiolabeling and immunoprecipitation, using methods described in, for example Harlow, et al., Antibodies: A Laboratory Manual, 2nd Ed., Cold Spring Harbor Laboratory Press, Cold Spring Harbor, New York (1988). To this end, two days after transfection, the cells are labeled by incubation in media containing 35S-cysteine for 8 hours. The cells and the media are collected, and the cells are washed and lysed with detergent-containing RIPA buffer: 150 mM NaCl, 1% NP-40, 0.1% SDS, 0.5% DOC, 50 mM TRIS, pH 7.5, as described by Wilson, et al. cited above. Proteins are precipitated from the cell lysate and from the culture media using an HA-specific monoclonal antibody. The precipitated polypeptides then are analyzed by SDS-PAGE and autoradiography. An expression product of the expected size is seen in the cell lysate, which is not seen in negative controls.

### Example 5

### Cloning and Expression in CHO Cells

The vector pC4 is used for the expression of monkey FLINT polypeptide. Plasmid pC4 is a derivative of the plasmid pSV2-dhfr (ATCC Accession No. 37146). The plasmid contains the mouse DHFR gene under control of the SV40 early promoter. Chinese hamster ovary- or other cells lacking dihydrofolate activity that are transfected with these plasmids can be selected by growing the cells in a selective medium (alpha minus MEM, Life Technologies) supplemented with the chemotherapeutic agent methotrexate. The amplification of the DHFR genes in cells resistant to methotrexate (MTX) has been well documented (see, e.g., F. W. Alt, et al., J. Biol. Chem. 253:1357-1370 (1978); J. L. Hamlin and C. Ma, Biochem. et Biophys. Acta 1097:107-143 (1990); and M. J. Page and M. A. Sydenham, Biotechnology 9:64-68 (1991)). Cells grown in increasing concentrations of MTX develop resistance to the drug by overproducing the target enzyme, DHFR, as a result of amplification of the DHFR gene. If a second gene is linked to the DHFR gene, it is usually co-amplified and over-expressed. It is known in the art that this approach can be used to develop cell lines carrying more than 1,000 copies of the amplified gene(s). Subsequently, when the methotrexate is withdrawn, cell lines are obtained which contain the amplified gene integrated into one or more chromosome(s) of the host cell.

Plasmid pC4 contains for expressing the gene of interest the strong promoter of the long terminal repeat (LTR) of the Rous Sarcoma Virus (Cullen, et al., Molec. Cell. Biol. 5:438-447 (1985)) plus a fragment isolated from the enhancer of the immediate early gene of human cytomegalovirus (CMV) (Boshart, et al., Cell 41:521-530 (1985)). Downstream of the promoter are BamHI, XbaI, and Asp718 restriction enzyme cleavage sites that allow integration of the genes. Behind these cloning sites the plasmid contains the 3' intron and polyadenylation site of the rat preproinsulin gene. Other high efficiency promoters can also be used for the expression, e.g., the human b-actin promoter, the SV40 early or late promoters or the long terminal repeats from other retroviruses, e.g., HIV and HTLVI. Clontech's Tet-Off and Tet-On gene expression systems and similar systems can be used to express the monkey FLINT in a regulated way in mammalian cells (M. Gossen, and H. Bujard, Proc. Natl. Acad. Sci. USA 89: 5547-5551 (1992)). For the polyadenylation of the mRNA other signals, e.g., from the human growth hormone or globin genes can be used as well. Stable cell lines carrying a gene of interest integrated into the chromosomes can also be selected upon co-transfection with a selectable marker such as gpt, G418 or hygromycin. It is advantageous to use more than one selectable marker in the beginning, e.g., G418 plus methotrexate.

The plasmid pC4 is digested with restriction enzymes and then dephosphorylated using calf intestinal phosphatase by procedures known in the art. The vector is then isolated from a 1% agarose gel.

The DNA sequence encoding the complete monkey FLINT polypeptide is amplified using PCR oligonucleotide primers corresponding to the 5' and 3' sequences of the gene. Non-limiting examples include 5' and 3' primers having nucleotides corresponding or complementary to a portion of the coding sequence of a monkey FLINT, e.g., as presented in at least one of SEQ ID NO:1, according to known method steps.

The amplified fragment is digested with suitable endonucleases and then purified again on a 1% agarose gel. The isolated fragment and the dephosphorylated vector are then ligated with T4 DNA ligase. *E.* coli HB101 or XL-1 Blue cells are then transformed and bacteria are identified that contain the fragment inserted into plasmid pC4 using, for instance, restriction enzyme analysis.

Chinese hamster ovary (CHO) cells lacking an active DHFR gene are used for transfection. 5 µg of the expression plasmid pC4 is cotransfected with 0.5 µg of the plasmid pSV2-neo using lipofectin. The plasmid pSV2neo contains a dominant selectable marker, the neo gene from Tn5 encoding an enzyme that confers resistance to a group of antibiotics including G418. The cells are seeded in alpha minus MEM supplemented with 1 µg/ml G418. After 2 days, the cells are trypsinized and seeded in hybridoma cloning plates (Greiner, Germany) in alpha minus MEM supplemented with 10, 25, or 50 ng/ml of methotrexate plus 1 µg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 mM, 2 mM, 5 mM, 10 mM, 20 mM). The same procedure is repeated until clones are obtained which grow at a concentration of 100 - 200 mM. Expression of the desired gene product is analyzed, for instance, by SDS-PAGE and Western blot or by reverse phase HPLC analysis.

### Example 6

### Tissue Distribution of Monkey FLINT mRNA Expression

Northern blot analysis is carried out to examine monkey FLINT gene expression in human tissues, using methods described by, among others, Sambrook, et al., cited above. A cDNA probe containing the entire nucleotide sequence of a monkey FLINT polypeptide (SEQ ID NO:1) is labeled with ³²P using the Rediprime™ DNA labeling system (Amersham Life Science), according to the manufacturer's instructions. After labeling, the probe is purified using a CHROMA SPIN-100™ column (Clontech Laboratories, Inc.), according to the manufacturer's protocol number PT1200-1. The purified and labeled probe is used to examine various human tissues for monkey FLINT mRNA.

Multiple Tissue Northern (MTN) blots containing various human tissues (H) or human immune system tissues (IM) are obtained from Clontech and are examined with the labeled probe using ExpressHyb hybridization solution (Clontech) according to manufacturer's protocol number PT1190-1. Following hybridization and washing, the blots are mounted and exposed to film at -70°C overnight, and films developed according to standard procedures. The results show monkey FLINT polypeptides to be selectively expressed in at least one of all or some tissues and other tissues.

### EXAMPLE 7

### Detecting Monkey FLINT interaction with FasL.

The binding between monkey FLINT and Fas L can be confirmed and the binding properties determined (e.g., kinetics, specificity, affinity, cooperativity, relative binding pattern) using real-time biomolecular interaction analysis (hereinafter BIA). To monitor biomolecular interactions, BIA uses optical phenomenon surface plasmon resonance. Detection of binding interactions depends on changes in the mass concentration of macromolecules at the biospecific interface. The following materials and methods were used.
Biacore® 2000 device (Biacore AB, Rapsgatan 7, S-754 50 Uppsala, Sweden)
Sensor Chip CM5 (Biacore)
Amine coupling kit (Biacore)
Washing buffer: HBS-EP (Biacore)
Guanidine Isothiocyanate Solution (GibcoBRL)
Fas L (Kamiya Biomedical Company, 910 Industry Drive, Seattle, WA 98188)
FLINT
Fas/Fc chimera (R&D Systems)

### Immobilization Protocol:

FasL or FLINT is immobilized through a primary amine group on lysine residues onto carboxymethyldextran polymer attached to a gold surface (Sensor Chip CM5). Immobilization is carried out using the amine coupling kit (Biacore) according to the manufacturer's protocol. Briefly, 100 µl of either a FasL or FLINT solution (10-25 µg/ml in sodium acetate buffer 20 mM, pH 5.0) is loaded onto an activated CM5 chip. After coupling, excess reactive groups on the surface are deactivated with 1M ethanolamine hydrochloride pH 8.5. The chip is then washed with a sodium acetate buffer 20 mM, pH 5.0 to remove non-covalently bound material.

### Interaction Analysis:

To analyze the interaction between FasL and FLINT, solutions containing the FLINT are passed over the chip with FasL immobilized thereon. The amount of FLINT associated with FasL is determined by measuring the surface plasmon resonance signal (in responce units, RU). Typically, FLINT solutions at different concentrations in HBS-ET buffer are loaded on the sensor chip for 10 minutes at a flow rate of 5 µl/min. The chip is then washed with HBS-ET buffer (10 mM Hepes, pH 7.4; 150 mM NaCl, 3 mM EDTA, 0.005% Surfactant P20) for 2 minutes at a flow rate of 5 µl/min. The bioactive surface of the chip is then regenerated by exposure to 0.8 M guanidine isothiocyanate for 2 minutes at a flow rate of 5 µl/min and then HBS-EP buffer for 2 minutes at 5 µl/min flow rate.

The converse reaction can also be done whereby a FasL solution is passed over a chip having FLINT immobilized thereon. The amount of FasL associated with FLINT is determined using surface plasmon resonance signal (in response units, RU).

### Determination of Affinity Constants:

The data is evaluated and binding parameters determined using BIA evaluation 3.0.2 software (Biacore). The Kd for the interaction between FLINT and FasL and between FasL and Fas is determined using the protocols described above.

### EXAMPLE 8

### FLINT and FLINT Variants Inhibit FasL-Induced Jurkat Cell Apoptosis

A bioassay measuring the prevention of apoptosis (i.e. cell survival) is performed using monkey FLINT and human FLINT variants (described in Example 9) produced in mammalian cell culture. For this purpose, 25 µl of Jurkat cells (5X10⁴ cells/well) is added to each well of a 96-well plate and mixed with 25 µl of recombinant human FasL (final concentration 150ng/ml), and either 50 µl of FLINT or FLINT variant. Serial dilutions ranging from 0 to 1 mg/ml are tested in the assay. Cells are incubated at 37°C over night. Twenty µl of MTS tetrazolium compound (Promega Corporation, Madison, WI) is added to each well and the incubation carried out for 2h at 37°C. Absorbance at 490 nm is recorded using a plate reader.

### Example 9

### Directed Mutagenesis Of Human FLINT Polypeptides

The polymerase chain reaction (PCR) can be used for the enzymatic amplification and direct sequencing of small quantities of nucleic acids (see, e.g., Ausubel, *supra,* section 15) to provide specified substitutions, insertions or deletions in DNA encoding a human FLINT polypeptide of the present inventions, e.g., SEQ ID NO:2, as presented herein, to provide a variant FLINT polypeptide sequence including at least one substitution, insertion or deletion selected from 10S,10T,10L,10V,10I,10G,10A,16I,16V, 16A,16G,16L,23L,23I,23V,23A,23G,23P,27V,27G,27A,27L,27I,30L, 30I,30V,30A,30G,31A,31G,31V,31L,31I,33S,33T,33A,33G,33L,33V, 33I,41T,41G,41A,41V,41L,41I,42E,42N,42Q,46R,46H,46K,60R,60K, 60H,104A,104G,104V,104L,104I,131S,131T,131I,133L,131A,131G,1 36A,136G,136V,136L,136I,139I,139L,139G,139A,139V,191T,191S,1 91A,191G,191L,191I,191V,198G,198A,198V,198I,198L,208A,208G,2 08L,208L,208V,208I,212E,212D,212N,212Q,238A,238G,238L,238V,2 381,254Q,254N,254D,254E,254R,254K,254H,258R,258K,258H,258Q,2 58N,258D,258E,259R,259K,259H,259Q,259N,259D,259E,266G,266A,2 66V,266L,266I,266D,266E,266N,266Q,299V,299L,299I,299A,299G, respectively, of SEQ ID NO:2. This technology can be used as a quick and efficient method for introducing any desired sequence change into a DNA of interest.

Unit 8.5 of Ausubel, *supra,* contains two basic protocols for introducing base changes into specific DNA sequences. Basic Protocol 1, (entirely incorporated herein by reference), describes the incorporation of a restriction site and Basic Protocol 2, as presented below and in the second section of Unit 8.5 of Ausubel, *supra,* details the generation of specific point mutations (all of the following references in this example are to sections of Ausubel et al., eds., Current Protocols in Molecular Biology, Wiley Interscience, New York (1987-1999)).

An alternate protocol describes generating point mutations by sequential PCR steps. Although the general procedure is the same in all three protocols, there are differences in the design of the synthetic oligonucleotide primers and in the subsequent cloning and analyses of the amplified fragments.

The PCR procedure described here can rapidly and efficiently introduce any desired change into a DNA fragment. It is similar to the oligonucleotide-directed mutagenesis method described in UNIT 8.1, but does not require the preparation of a uracil-substituted DNA template.

The main disadvantage of PCR-generated mutagenesis is related to the fidelity of the Taq DNA polymerase. The mutation frequency for Taq DNA polymerase was initially estimated to be as high as 1/5000 per cycle (Saiki et al., 1988). This means that the entire amplified fragment must be sequenced to be sure that there are no Taq-derived mutations. To reduce the amount of sequencing required, it is best to introduce the mutation by amplifying as small a fragment as possible. With rapid and reproducible methods of double-stranded DNA sequencing, the entire amplified fragment can usually be sequenced from a single primer. If the fragment is somewhat longer, it is best to subclone the fragment into an M13-derived vector, so that both forward and reverse primers can be used to sequence the amplified fragment.

If there are no convenient restriction sites flanking the fragment of interest, the utility of this method is somewhat reduced. Many researchers prefer the mutagenesis procedure in UNIT 8.1 to avoid excessive sequencing.

A full discussion of critical parameters for PCR amplification can be found in UNIT 15.1.

It will be clear that the invention can be practiced otherwise than as particularly described in the foregoing description and examples. Numerous modifications and variations of the present invention are possible in light of the above teachings and, therefore, are within the scope of the appended claims.

## Claims

1. An isolated nucleic acid that encodes a protein having the sequence of SEQ ID NO:4.

2. An isolated nucleic acid encoding a FLINT protein having at least one substitution in SEQ ID NO:2 selected from the group consisting of:
a) Ser at position 10 is alternatively substituted by Thr, Leu, Val, Ile, Gly, or Ala;
b) Leu at position 16 is alternatively substituted by Ile, Val, Ala, Gly;
c) Pro at position 23 is alternatively substituted by Leu, Ile, Val, Ala, Gly;
d) Val at position 27 is alternatively substituted by Gly, Ala, Leu, Ile;
e) Val at position 30 is alternatively substituted by Leu, Ile, Val, Ala, Gly;
f) Ala at position 31 is alternatively substituted by Gly, Val, Leu, Ile;
g) Thr at position 33 is alternatively substituted by Ser, Ala, Gly, Leu, Val, Ile;
h) Ala at position 41 is alternatively substituted by Thr, Gly, Val, Leu, Ile;
i) Glu at position 42 is alternatively substituted by Asn, Gln;
j) Arg at position 46 is alternatively substituted by His, Lys;
k) Arg at position 60 is alternatively substituted by His, Lys;
l) Ala at position 104 is alternatively substituted by Gly, Val, Leu, Ile;
m) Ser at position 131 is alternatively substituted by Thr, Ile; Leu, Ala, Gly;
n) Ala at position 136 is alternatively substituted by Gly, Val, Leu, Ile;
o) Ile at position 139 is alternatively substituted by Leu, Gly, Ala, Val;
p) Thr at position 191 is alternatively substituted by Ser, Ala, Gly, Leu, Ile, Val;
q) Gly at position 198 is alternatively substituted by Ala, Val, Ile, Leu;
r) Ala at position 208 is alternatively substituted by Gly, Leu, Val, Ile;
s) Glu at position 212 is alternatively substituted by Asp, Asn, Gln;
t) Ala at position 238 is alternatively substituted by Gly, Leu, Val, Ile;
u) Gln at position 254 is alternatively substituted by Asn, Asp, Glu, Arg, Lys, His;
v) Arg at position 258 is alternatively substituted by Lys, His, Gln, Asn, Asp, Glu;
w) Arg at position 259 is alternatively substituted by Lys, His, Gln, Asn, Asp, Glu;
x) Gly at position 266 is alternatively substituted by Ala, Val, Leu, Ile, Asp, Glu, Asn, Gln; and
y) Val at position 299 is alternatively substituted by Leu, Ile, Ala, Gly.

3. A recombinant vector, comprising at least one nucleic acid according to claim 1 or claim 2.

4. A host cell comprising at least one recombinant vector according to claim 3.

5. A method for producing a recombinant FLINT polypeptide comprising culturing a host cell according to claim 4 under conditions that allow expression of said polypeptide in detectable or recoverable amounts.

6. An isolated FLINT polypeptide variant of SEQ ID NO:2 said polypeptide comprising an amino acid substitution selected from the group consisting of:
a) Ser at position 10 is alternatively substituted by Thr, Leu, Val, Ile, Gly, or Ala;
b) Leu at position 16 is alternatively substituted by Ile, Val, Ala, Gly;
c) Pro at position 23 is alternatively substituted by Leu, Ile, Val, Ala, Gly;
d) Val at position 27 is alternatively substituted by Gly, Ala, Leu, Ile;
e) Val at position 30 is alternatively substituted by Leu, Ile, Val, Ala, Gly;
f) Ala at position 31 is alternatively substituted by Gly, Val, Leu, Ile;
g) Thr at position 33 is alternatively substituted by Ser, Ala, Gly, Leu, Val, Ile;
h) Ala at position 41 is alternatively substituted by Thr, Gly, Val, Leu, Ile;
i) Glu at position 42 is alternatively substituted by Asn, Gln;
j) Arg at position 46 is alternatively substituted by His, Lys;
k) Arg at position 60 is alternatively substituted by His, Lys;
l) Ala at position 104 is alternatively substituted by Gly, Val, Leu, Ile;
m) Ser at position 131 is alternatively substituted by Thr, Ile; Leu, Ala, Gly;
n) Ala at position 136 is alternatively substituted by Gly, Val, Leu, Ile;
o) Ile at position 139 is alternatively substituted by Leu, Gly, Ala, Val;
p) Thr at position 191 is alternatively substituted by Ser, Ala, Gly, Leu, Ile, Val;
q) Gly at position 198 is alternatively substituted by Ala, Val, Ile, Leu;
r) Ala at position 208 is alternatively substituted by Gly, Leu, Val, Ile;
s) Glu at position 212 is alternatively substituted by Asp, Asn, Gln;
t) Ala at position 238 is alternatively substituted by Gly, Leu, Val, Ile;
u) Gln at position 254 is alternatively substituted by Asn, Asp, Glu, Arg, Lys, His;
v) Arg at position 258 is alternatively substituted by Lys, His, Gln, Asn, Asp, Glu;
w) Arg at position 259 is alternatively substituted by Lys, His, Gln, Asn, Asp, Glu;
x) Gly at position 266 is alternatively substituted by Ala, Val, Leu, Ile, Asp, Glu, Asn, Gln; and
y) Val at position 299 is alternatively substituted by Leu, Ile, Ala, Gly.

7. A composition, comprising at least one isolated polypeptide according to claim 6 and a carrier or diluent.

8. An antibody or fragment thereof that binds an epitope specific to a monkey FLINT polypeptide defined herein by SEQ ID NO:4.

9. A method for identifying compounds that bind a monkey FLINT polypeptide, comprising
(a) admixing at least one isolated monkey FLINT polypeptide with at least one test compound or composition; and
(b) detecting at least one binding interaction between said at least one monkey FLINT polypeptide and the test compound or composition.

10. A compound or composition detected by method according to claim 9.

11. A method for the production of a pharmaceutical composition comprising identifying the compound according to the method of Claim 9, synthesising the compound and optionally admixing with a pharmaceutically acceptable carrier or diluent.
